(19) Europäisches Patentamt / European Patent Office / Office européen des brevets

(11) **EP 3 335 536 B1**

(12) **FASCICULE DE BREVET EUROPEEN**

(45) Date de publication et mention de la délivrance du brevet:
**17.01.2024  Bulletin 2024/03**

(21) Numéro de dépôt: **17196251.7**

(22) Date de dépôt: **12.10.2017**

(51) Classification Internationale des Brevets (IPC):
**A01C 21/00** (2006.01)   **C12Q 1/02** (2006.01)
**G01N 33/24** (2006.01)

(52) Classification Coopérative des Brevets (CPC):
**G01N 33/24;** A01C 21/00; G01N 2033/245

(54) **FERTILISATION AZOTOBACTÉRIENNE RAISONNÉE (FAR)**

AUSGEWOGENE AZOTOBAKTERIELLE DÜNGUNG

RATIONED AZOTOBACTERIAL FERTILISATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.12.2016  FR 1670757**

(43) Date de publication de la demande:
**20.06.2018  Bulletin 2018/25**

(73) Titulaire: **Polyor SARL**
**54000 Nancy (FR)**

(72) Inventeur: **Claude, Pierre-Philippe**
**54000 Nancy (FR)**

(56) Documents cités:
**EP-A1- 2 845 906**   WO-A1-2016/172582
**FR-A1- 2 910 230**   US-A- 5 668 719

• TIAN XIAO-FANG ET AL: "Influence of nitrogen fertilization on soil ammonia oxidizer and denitrifier abundance, microbial biomass, and enzyme activities in an alpine meadow", BIOLOGY AND FERTILITY OF SOILS, BERLIN, DE, vol. 50, no. 4, 30 novembre 2013 (2013-11-30), pages 703-713, XP035313765, ISSN: 0178-2762, DOI: 10.1007/S00374-013-0889-0 [extrait le 2013-11-30]

**Description**

**DOMAINE TECHNIQUE DE L'INVENTION**

La fertilisation raisonnée, notamment en azote, mais aussi par azotobactérisation des résidus de culture au sol, y compris par inoculation, et plus généralement ladite biofertilisation microbiennes à l'aide microorganismes phytogènes.

**ÉTAT DE LA TECHNIQUE**

Bien que la méthode Comifer (2013) - dite du bilan, pour le calcul des doses prévisionnelles d'engrais azoté (dX) fasse l'objet d'un consensus, cette approche est aujourd'hui controversée (Ravier et al. 2015, 2016, 2017). De plus, cette méthode de *fertilisation raisonnée* (FR) ne comptabilise pas véritablement l'activité des azotobactéries telluriques, notamment en présence de *résidus de culture au sol* (RCS) ; les gains d'N via la *fixation non symbiotique* ($F_{ns}$) ne ferraient que compenser les pertes gazeuses d'N par *dénitrification* ($G_s$), le bilan de la $F_{ns}$ étant en ce sens nul ou négligeable. Les nombreux postes (eg. reliquats sortie-hiver (RSH) d'azote minéral (Nm), minéralisation, immobilisation et dénitrification de l'azote du sol, etc.) sont additifs, ce qui n'est pas nécessairement le cas in situ puisque ces processus sont simultanées et fortement interdépendant, les erreurs associées à chaque poste vont s'accumuler. Enfin, Ravier et al. 2015 notèrent que les analyses de sol devant en principe améliorer la précision de dX, notamment les RSH, sont en elles même sources d'erreur.

Plus particulièrement, il y a aussi la question de la minéralisation de l'azote organique du sol (Mh). L'analyse de 130 parcelle dans l'ouest de la France (Morvan et al. 2015) montra que la minéralisation nette mesurée au champ ne peut dans certaines conditions s'expliquer que par la prise en compte d'un processus d'*extra-minéralisation* (sic). In fine, ces méthodes dépendent trop de mesures intra-saisonnières telles que les RSH, ou encore de l'indicateur dynamique de la de nutrition azotée (NNI ; Justes et al. 1994). Ces mesures entraînent l'engorgement des laboratoires d'analyses (RSH) et/ou des imprécisions du fait qu'il s'agit de mesures qu'indirectes de l'état azotée de la plante (NNI). Ravier et al. 2015 concluent donc qu'il faut trouver d'autres moyens d'estimer dX, par exemple la modélisation et/ou des méthodes FR n'impliquant pas dX (Ravier et al. 2017, Arvalis 2016) - quitte à faire fi de la notion de potentiel (objectif) de rendement, en effectuant un suivi plus tenu d'NNI (indice de nutrition azoté ; Justes et al. 1994).

*L'analyse multicritère agroenvironnementale*

Andrews et al. 2002/2004 et Glover et al. 2000 décrivent des méthodes de traitement de données capables d'indiquer la qualité ou encore la durabilité de sols arables. Il s'agit d'agréger divers *indicateurs élémentaires* en un seul indicateur, dit agrégé. Les *indicateurs élémentaires* (Lairez et al. 2015) sont des variables qui synthétisent ou simplifient les données brutes pertinentes relatives à l'état ou à l'évolution d'un phénomène. Les données brutes sont des mesures ou des descriptions de phénomènes ou de pratiques (agricoles), et prennent le statut d'indicateur à partir du moment où elles sont comparées à des valeurs de références. Les *indicateurs agrégés* (Lairez et al. 2015) sont des variables résultant de l'agrégation d'indicateurs élémentaires d'un même phénomène ou critère, et exprimés dans une unité commune (score) avant d'être agrégés. Ici, cette agrégation se fait par sommation de scores normalisés et pondérés selon leur contribution à une espace multi-vectorielle. Selon Boukherroub et al. 2012, il existe deux méthodes d'agrégations d'indicateurs élémentaires ; (i) l'analyse multivariée issue des sciences statistiques (Lebart et al. 2000), et (ii) les méthodes d'aide à la décision multicritères (Saaty 2008). Or, comme leur nom l'indique, ces analyses multicritères présupposent qu'il existe de nombreuses données APC (agro-pédo-climatiques) provenant d'une même parcelle agricole.

*Azotobactérisation des résidus de culture au sol (RCS) et fertilisation raisonnée*

Les azotobactéries non symbiotiques ont longtemps été confinées aux seules *Azotobacteracea.* Or, le potentiel azotobactérien du sol est bien supérieur aux quelques kg-N/ha/an rapportés. Pourtant, la valorisation des RCS comme substrats carbonés pour de telles azotobactéries n'est toujours pas une pratique culturale très répandue. Pour causes. En effet, et malgré son potentiel agronomique, l'azotobactérisation des RCS demeure parfois contreproductive (Claude et Fillon 2004) du fait d'une certaine *surimmobilisation* de l'azote minéral (EP2223586) et d'une fertilisation raisonnée inadaptée comportant un rationnement arbitraire, voire aveugle, de dX. En effet, et bien que l'efficacité de telles azotobactérisations (eAZB) des RCS soit affectée par la quantité d'azote minéral (Nm) résiduel rétroagissant négativement sur la diazotrophie azotobactérienne, un rationnement aveugle de dX ne tenant pas compte de cette surimmobilisation de l'azote peut paradoxalement surtout nuire à l'alimentation en N de la culture. A défaut d'une FR adaptée à de telles azotobactérisations, il risque donc d'avoir trop, ou trop peu, d'N dans le système sol-plante pour que lesdites azotobactéries puissent fonctionner optimalement. La préconisation de tels engrais ne peut donc pas faire fi d'eAZB. Certaines méthodes de FR sont connues de US 5 668 719 A et EP 2 845 906.

Pourtant, certains laboratoires proposent des indicateurs d'activité de la vie microbienne des sols en principe applicable à de telles azotobactérisations. Ces indicateurs sont soit trop (i) monocritères et/ou insuffisamment pondérés, (ii) trop axés sur la notion d'activité de la flore microbienne du sol dans son ensemble plutôt que sur celles des azotobactéries résidusphériques, et/ou (iii) logistiquement complexes.

Sigles et Définitions

**AZB** : azotobactérisation des résidus de culture au sol (RCS)
**RCS** : résidus de culture (cellulosiques) au sol
**FR** : fertilisation raisonnée
**FAR** : fertilisation azotobactérienne raisonnée
**eAZB** : efficacité de l'azotobactérisation, y compris par inoculation, des RCS par rapport à un témoin
**eMIC** : idem, mais de microorganismes apportés au système sol-plant à titre de de biofertilsiation
**pAZB** : potentiel azotobactérien d'un sol, notamment en présence de RCS, affectant eAZB
**pMIC** : potentiel microbienne d'un sol, appréciable via et affectant eMIC
**iAZB** : indicateur agrégé d'indicateurs élémentaires affectant eAZB
**iMIC** : indicateur agrégé d'indicateurs élémentaires affectant eMIC
**FAS** : fonction d'attribution de scores (normalisés), ou *fonction d'utilité*
**RDT** : rendement agronomique, généralement ici en quintaux (Qx) par hectare (Qx/ha)
**RDN** : rendement protéique en kg-N, ou de protéine selon le cas, par hectare = RDT x % protéine du grain
**RUN** : rendement unitaire, soit en RDT ou RDN par uN (Nb. uN = 1 kg-N$_{fertilisant}$ par hectare).
**RAT** : reliquats automnaux d'azote minéral (Nm) du sol (uN)
**RSH** : reliquats sortie-hiver d'azote minéral (Nm) du sol (uN ; cf Comifer 2013)
**RPR** : reliquats post-récolte d'azote minéral (Nm) du sol (uN)
**iAZK** / EP13366004.3 / EP2684588 - kit d'échantillonnage de terre comportant un filtre macro-microporeux constitué d'un feuilleté multi-plis semi-rigide ou souple avec 4 couches, (i) la première n'étant pas en contact avec l'échantillon est étanche, (ii) la deuxième immédiatement sous la première est microporeuse ne permettant pas le passage des bactéries, (ii) la troisième immédiatement sous la deuxième est macroporeuse, et (iv) la quatrième immédiatement sous la troisième est elle aussi microporeuse.
**iAPC** / EP13366005.0 / EP2728353 - indicateur élémentaire d'eMIC pour l'affectation d'un agro/micro - pédoclimat (APC) à une parcelle et/ou un îlot agricole par transcription de variables pédologiques descriptives et d'un échantillon de terre arable en variables agro/micro-pédologiques comprenant (i) la classification des échantillons sur la base de leurs teneurs en argile, et (ii) la segmentation de ces classes en APC. Ce procédé est caractérisé en ce qu'il permet la formation de consortia (azoto)bactériens biofertilisants APC spécifiques.
**iRES** / EP13366006.8 / EP2730926 - indicateur élémentaire d'eMIC pour évaluation de la résilience des bactéries d'un sol en termes de diversité, d'activité et/ou d'abondance confrontées à des variations de pH comprenant une mesure de la dynamique de la fraction bactérienne du sol à différent pH comprenant l'étendue de l'acidité échangeable du sol et caractérisée en ce que la résilience bactérienne est appréciée par rapport au pH optimal (pHo) en termes de diversité, d'activité et/ou d'abondance de cette microflore.
**iREC** / EP14290257.6 / EP2845906 - indicateur élémentaire d'eMIC pour l'évaluation de l'efficience carbonée (eC) de la microflore tellurique selon la quantité de reliquats automnaux d'Nm (RAT) et de préconisation de de l'azotobactérisation, y compris par inoculation, des RCS via un indicateur de l'eC bactérienne (BAC) et microbienne (MIC), $eC_{BAC}/eC_{MIC}$, comprenant les RAT et le ratio N:C ($rb_{MIC}$) des microorganismes du sol (MIC), le ratio C/N ($rCN_{sol}$) et le ratio des fractions fongique et bactérienne du sol (rFB).
**iDAM** / EP15290111.2 / EP3120679 - indicateur élémentaire d'eMIC pour le diagnostic de l'état diazotrophe de sols et de préconisation de la fertilisation de la culture par rationnement de la dose X (dX) d'engrais azoté (N) préalablement calculée comportant qu'un seul relevé des RAT - sans échantillonner l'ensemble de la zone d'enracinement et/ou de la zone vadose, et un indicateur constitué de la somme d'unités de temps post - enfouissement des RCS proportionnel au niveau d'activité diazotrophe du sol.
**iVAZ** / EP16179846.7 / EP3120679 - indicateur élémentaire pour la fertilisation raisonnées par (i) détermination de la quantité de RCS, (ii) échantillonnage représentatif et (iii) détermination de la teneur en N de ces RCS (Nrcs), ainsi que l'obtention des valeurs RVN = [ RCS / Nrcs ] et rMS = [ RCS / RAC ], RAC étant la biomasse racinaire. rNS, fonction de RVN, est le ratio Nrcs/Nrac, et iVAZ = [ rMS - rNS ]. Si iVAZ est inférieur à une certaine valeur critique, le rationnement de la dose X n'est pas préconisé.

**iPAZ /** EP16179850.9 / EP3120680 - Calibration d'engrais P aux cultures agronomiques une courbe de réponse mitscherlichienne de l'activité diazotrophe des azotobactéries en sols arables en proximité des RCS fonction, (i) des teneurs en C, P, voire N, desdit résidus de culture cellulosiques, avantageusement pailleux, au sol (RCS) avant leur enfouissement en pré - semis d'une culture agronomique suivante et, (ii) d'un équivalent de phosphore exogène, dP, proportionnel aux susdites teneurs en C et P des RCS.

**Indicateurs élémentaires** (cf. Lairez et al. 2015) : utilisés quand un phénomène ne peut pas être décrit directement, ou précisément, en raison de sa complexité. Ils fournissent des informations sur un phénomène en le quantifiant ou le qualifiant. Ils simplifient l'information en se basant sur des observations, des données, des sorties de modèles, ou des mesures. Ils permettent par exemple de mesurer les critères du développement durable, ou plus nouvellement ici, de la FAR.

**Indicateurs agrégés** (cf. Lairez et al. 2015) : résultent de l'agrégation de plusieurs indicateurs élémentaires impliqués dans un même phénomène, ces indicateurs élémentaires étant exprimés dans une unité commune ou un score (Si) avant d'être agrégés. Cette dernière repose, soit sur une relation empirique démontrée (eg. eAZB :: iPAZ, iREC, etc.), soit sur des conventions établies par des experts définissant ce qui compose un tel critère élémentaire.

**Résidusphère** : Volume ou zone du sol entourant immédiatement les résidus de culture pailleux, engrais organo-minéraux et/ou biomasses racinaires résiduelles, et plus ou moins influencé par ceux-ci selon leur distance de ces substrats métaboliques, soit généralement inférieure à 4 mm. Il est aussi question d'un « noyau » (*hotspots*) où se situe la décomposition desdits résidus de culture.

**Microorganismes phytogènes** (*plant growth promoting microorganisms*) : Microorganismes, généralement d'origine telluriques, capables de promouvoir la croissance et/ou le développement des plantes. Ceux-ci peuvent être en symbiose ou en association avec les racines, voire ici plus en interactions plus lâches avec la résidusphère issue de l'enfouissement et la dégradation in situ de résidus de culture.

## DIVULGATION DE L'INVENTION

*Problème technique*

La FR via la méthode du bilan (Comifer 2013) et le pilotage intra-saisonnier des apports fractionnés d'engrais N sont des imparfaits et controversés. Cela dit, les susdits indicateurs agrégés de la qualité des sols ou durabilité des agroécosystèmes (Knoepp et al. 2000, Schloter et al. 2003, Glover et al. 2000, Bastida et al. 2008, Horwath et al. 2002, etc.) ne sont pas suffisamment quantifiables ou précis pour être directement applicables au calcul des doses prévisionnelle d'engrais N. De plus, l'actuelle fertilisation raisonnée en azote selon la méthode du bilan ne tient pas compte adéquatement de l'activité des azotobactéries (diazotrophie) du sol-notamment en présence de RCS.

*Solution technique (évaluation de l'efficacité microbiennne, eMIC)*

La solution technique contient un élément nouveau et inventif, à savoir l'utilisation de manière quantitative d'indicateurs agrégés d'eMIC ou d'eAZB pour l'estimation précise de la dose prévisionnelle d'engrais N. Les indicateurs élémentaires ainsi agrégés et provenant de données d'échantillons terre permettent de calculer les doses NP prévisionnelles sans recourir aux habituelles mesures de reliquats (d'azote minéral) sortie hiver (RSH), de potentiel de minéralisation de la matière organique du sol (Mh), voire l'ensemble des autres postes de l'équation du bilan (Comifer 2013).

Concrètement, il s'agit d'une méthode de fertilisation-N raisonnée pour grandes cultures agronomiques selon la revendication 1. Cette méthode comporte la formation, la calibration, la préconisation et l'épandage au champs de doses d'engrais N prévisionnelles et comprend les étapes suivantes ;

- la détermination de **$n$ indicateurs élémentaires** de l'**efficacité microbienne (eMIC)** de microorganismes phytogènes du système sol-plant, y compris ceux réintroduits par inoculation, à augmenter les rendements agronomiques (RDT), protéiques (RDN) ou unitaires (RUN) de la culture agronomique par rapport à un témoin,
- l'agrégation de ces n indicateurs (i) élémentaires d'eMIC, après leurs transformations en autant de scores normalisés et pondérés, en un **indicateur agrégé (iMIC)** provenant donc de l'agrégation de ces n indicateurs élémentaires obtenus des données pédoclimatiques de la couche arable,
- mise en relation d'eMIC avec iMIC,
- la calibration de ces doses d'engrais-N (**$dN$**) est fonction d'eMIC ainsi déduites empiriquement d'iMIC,
- pesée, préconisation et épandage des engrais N correspondant à dN dans laquelle; l'agrégation des indicateurs élémentaires en indicateurs agrégés, par exemple ici *iMIC,* ce fait somme suit ;

$$iMIC = \sum(Si \times Pi) \text{ pour les } i = 1, 2, 3 \ldots \rightarrow \text{nombre d'indicateurs } (n)$$

Si étant ici le score normalisé (base 100 ou 1,00) de l'indicateur élémentaire affectant eMIC, et *Pi* le poids (0,00 à 1,00) attribué à ce score, pMIC est ici en sorte la contribution (fourniture) en N des microorganismes phytogènes du sol, y compris ceux réintroduits par l'inoculation des RCS.

La préconisation et l'application pondérale in situ précises de la dose d'engrais N (dN) en fonction de l'indicateur agrégé, iMIC, lui-même obtenu par agrégation des indicateurs élémentaires de la capacité des microorganismes phytogènes du sol, y compris ceux réintroduits par inoculation au système sol - plant, ou encore stimulés in situ par application de molécules signalétiques ou bio-stimulantes, à augmenter RDN et RUN étant calculé comme suite ;

$$dN = a \times pRDT - pMIC$$

sachant que ;

- $a = b_{ARVALIS} / eMIC_{RUN}$
- $pMIC = pRDN - pRDN/eMIC_{RDN}$

et que pRDT et pRDN sont les objectifs, ou potentiels, de rendements agronomiques ($Qx_{grain}$/ha) et protéiques (kg-$N_{grain}$/ha), $eMIC_{RDN}$ et $eMIC_{RUN}$ les valeurs d'eMIC en termes de RDN et RUN (rendements unitaires ; kg-$N_{grain}$/unité d'N fertilisant), respectivement et par rapport à un témoin non traité, et que a est le coefficient b dit d'Arvalis™ ($b_{ARVALIS}$), alias « besoin unitaire » (i.e. unités d'N par $Qx_{grain}$), divisée par $eMIC_{RUN}$. Pour ce qui est du potentiel, ou objectif de rendement. Bien que sont utilisation dans le calcul de dX, ou dN, soit controversé (Arvalis 2016, Ravier et al. 2015, 2016 et 2017), cette valeur me semble néanmoins toujours défendable puisqu'elle s'appuie sur des moyennes quinquennale ou décanales, et peut être par la suite actualisée en temps réel selon la météorologie, au niveau de la parcelle, de la commune, voire du département (eg. iDAM).

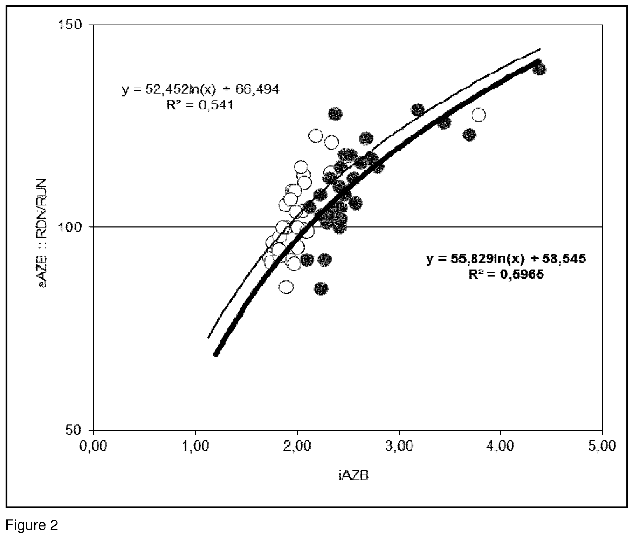

pMIC est ici en sorte la contribution (fourniture) en N des microorganismes résidusphériques phytogènes, y compris ceux réintroduits par l'inoculation. La formation de la dose d'engrais N (dN), et P le cas échéant, à partir de matières fertilisantes connues, sa préconisation sous forme d'apports fractionnés, et son épandage au champ étant eux effectués de manières conventionnelles. Dans le faits, eMIC va donc progresser selon iMIC (eg. Figure 2 pour le cas d'eAZB - iAZB, infra). Ce mode d'agrégation et de pondération d'indicateurs élémentaire normalisés en indicateur agrégés a été mise en oeuvre par Andrews et al. 2002/2004 et Glover et al. 2000. Pour ce qui est ici du cas eAZB - iAZB, la pondération des Si (Tableau 6) est ici effectuée à l'aide des valeurs propres au Tableau 4 selon Lairez et al. 2015.

Les indicateurs élémentaires d'eMIC agrégés en indicateurs agrégés ou composites obtenus par échantillonnage de la couche arables de parcelles agricoles sont choisi parmi un groupe comprenant ;

- iAPC, i.e. un indicateur élémentaire du niveau de spécificité agropédoclimatique des biomasses azotobactérienne d'un échantillon de terre provenant d'une à une parcelle agricole ;
- iRES, i.e. un indicateur élémentaire du niveau de résilience de la microflore bactérienne d'un sol en termes de diversité, d'activité et/ou d'abondance confrontée à des variations de pH du sol ;
- iREC, i.e. un indicateur élémentaire de l'efficience carbonée (eC) de la microflore tellurique, notamment azotobactérienne, en proximité des RCS ;
- iDAM, i.e. un indicateur élémentaire de l'état diazotrophe du sol comportant qu'un relevé des RAT et un indicateur d'activité diazotrophe des bactéries du sol.
- iVAZ, i.e. un indicateur élémentaire de l'activité des azotobactéries du sol intégrant la quantité de RCS et leurs teneurs en N, ainsi que les valeurs RVN = [ RCS / Nrcs ] et rMS = [ RCS / RAC ] ;
- iPAZ, i.e. un indicateur élémentaire de la quantité d'engrais P nécessaire à la flore azotobactérienne résidusphérique pour fonctionner de manière optimale en termes agronomique.

La transformation les n différents indicateurs élémentaires (i) d'eMIC en autant de scores normalisés (Si) ce fait à l'aide de n fonctions d'attribution de scores (*alias* FAS, ou fonction d'utilités) décrivant ces indicateurs élémentaires corrélés avec l'efficacité microbienne (eMIC) de microorganismes phytogènes du système sol-plant, y compris ceux réintroduits par inoculation, à augmenter les rendements agronomiques (RDT), protéiques (RDN) ou unitaires (RUN) de la culture par rapport à un témoin dépourvu de ces mêmes microorganismes phytogènes. Plus particulièrement, cette transformation en score normalisé (Si) des différents indicateurs élémentaires permettant d'exprimer ces différents indicateurs élémentaires sur une même échelle ou dans une même unité et consistant à obtenir des classes, scores ou variables continues à partir de ces indicateurs élémentaires et de leurs valeurs de référence, est effectuée par (cf. Lairez et al. 2015) ;

- classement

- notations discrètes sur une échelle fixe

- notations discrètes sur une échelle variable

- notations sur une échelle continue

- transformation en une unité de mesure commune

- normalisation interne.

Cette transformation en score normalisé (Si) des différents indicateurs élémentaires est calculé par rapport à une valeur choisie parmi un groupe non exhaustif comprenant ;

- à la valeur minimale ou maximale de l'indicateur avant normalisation,
- à la moyenne arithmétique, géométrique ou ordonnée de l'indicateur avant normalisation,
- à l'intégrale de Choquet ou la valeur de Sharpley associées à l'indicateur avant normalisation.

De même, le poids, la pondération en sorte, Pi, attribuée aux scores normalisés (Si) est calculé par une étape au cours de laquelle des poids sont attribués aux indicateurs élémentaires à agréger. Plus particulièrement, cette pondération, Pi, attribuée aux scores normalisés (Si) est calculé selon d'une des méthodes de calcul choisie parmi un groupe non exhaustif comprenant (i) l'ACP (analyse par composante principale), (ii) l'AHP (*analytical hierarchy process),* (iii) l'opinion des acteurs de la fertilisation raisonnée, ou encore toutes autres méthodes statistiques ou de calcul qui de manière générale permettent la décomposition aux valeurs singulières par réduction d'un tableau comportant *x* variables sur *y* observations individuelles. Notons que plus la valeur propre, au sens de ces méthodes statistiques, d'un indicateur est importante, plus sa contribution à iMIC sera importante.

Notons le cas particulier, mais à ce jour le avantageux, lorsque eMIC est **eAZB,** et que pMIC est **pAZB,** permettant ainsi la détermination d'**iAZB** en présence de RCS azotobactérisés, y compris par inoculation et réintroduction d'azotobactéries s'il le faut, de manière à ce que les valeurs d'eAZB estimées directement d'iAZB permettent elles aussi d'estimer dN comme suit ;

$$dN = \mathbf{a} \times pRDT - pAZB$$

sachant que a = [$b_{Arvalis}$ / $eAZB_{RUN}$] et pAZB = [pRDN - pRDN/$eAZB_{RDN}$]. pRDT et pRDN sont, respectivement, les objectifs, ou potentiels, de rendements agronomiques ($Qx_{grain}$/ha) et protéiques (kg-$N_{grain}$/ha), et $eAZB_{RDN}$ et $eAZB_{RUN}$ les valeurs d'eAZB fonction de RDN et RUN (rendements unitaires ; kg-Ngrain/uN fertilisant), *a* est le coefficient Arvalis ($b_{ARVALIS}$), alias « besoin unitaire » (uN/$Qx_{grain}$), divisé par $eAZB_{RUN}$, et pAZB représentent ici la contribution (ou fourniture) en N des azotobactéries résidusphériques, y compris celles réintroduites par l'inoculation des RCS, la formation de la dose d'engrais N et/ou P (dN) à partir de matières fertilisantes connues, sa préconisation sous forme d'apports fractionnés, et son épandage au champ étant eux pour le reste effectués de manières conventionnelles. Connaissant eAZB et pAZB via iAZB, nous pouvons ajuster dN soit en modulant les besoins unitaires ($\rightarrow$ a) en fonction d'd'$eAZB_{RUN}$, soit en modulant la contribution en N des azotobactéries ($\rightarrow$ pAZB) en fonction d'$eAZB_{RDN}$. Notons cependant que dN ne sera pas nécessairement inférieure à dX. Au contraire.

Enfin, les engrais N concernes par la présente invention peuvent être avantageusement des engrais à libération contrôlée (ELC, ou en anglais CRF ; *controlled release fertilizers),* ou encore des engrais à libération, ou dissolution, lente (EDL, ou en anglais SRF ; *slowrelease fertilizers).*

*Activité inventive et avantages apportés*

L'utilisation de tels indicateurs agrégés (iAZB) pour la détermination d'eAZB et le calcul de dN est nouvelle. De tels indicateurs ont été proposés pour l'évaluation multicritère de la durabilité d'agrosystèmes (Lairez et al. 2015) ou la qualité de sols arables (Schloter et al. 2003), mais jamais pour l'ajustement précis des doses prévisionnelles d'engrais azotés. Ce nouveau concept de FAR n'abandonne pas pour autant la notion d'objectif de rendements au profit d'un suivi plus tenu de l'indice de nutrition azotée (INN ; Arvalis 2016), et n'entraîne aucune augmentation de la charge de travail de l'utilisateur. Appliqué à d'autres types de biofertilisations (PGPR, mycorhizes, Penicillium, etc.), nous pouvons aussi envisager des dN calibrées selon eMIC, expressément selon chacune de ces technologies. Enfin, la FAR est beaucoup plus simple à réaliser que la méthode du bilan dans la mesure où les RCS sont azotobactérisés de manière à sur-immobiliser les reliquats d'azote minéral automnaux (RAT), ces derniers étant maintenant beaucoup plus déterminant des rendements agronomiques.

## BRÈVE DESCRIPTION DES DESSINS ET FIGURES

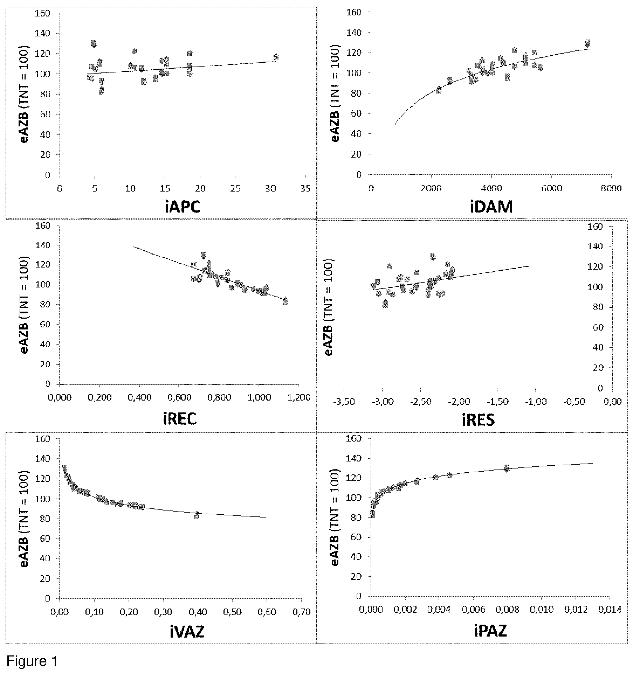

**Figure 1** : Exemples de fonctions d'attribution de scores (FAS) d'indicateurs élémentaires normalisés sur une base 1,00 = valeurs maximales pour ces six (6) indicateurs élémentaires d'eAZB. Ces scores normalisés (Si ; Tableaux 3 et 6) sont plus ou moins corrélés avec l'efficacité de l'azotobactérisation (eAZB) des RCS par rapport à un témoin non traité (TNT = 100%) en termes de rendement protéique (RDN ; kg-$N_{grain}$/ha).

**Figure 2** : Progression d'eAZB en termes de rendement protéique (RDN ; kg de protéine dans le grain / hectare) et unitaire (RUN ; kg de protéine dans le grain / unité d'azote fertilisant) du blé tendre d'hiver (BTH) selon la valeur de l'indicateur agrégé iAZB. La valeur d'iAZB est à ce stade putative et n'intègre que les indicateurs iAPC, iDAM, iRES, iREC, iVAZ et iPAZ tels que décrit et référencés ici.

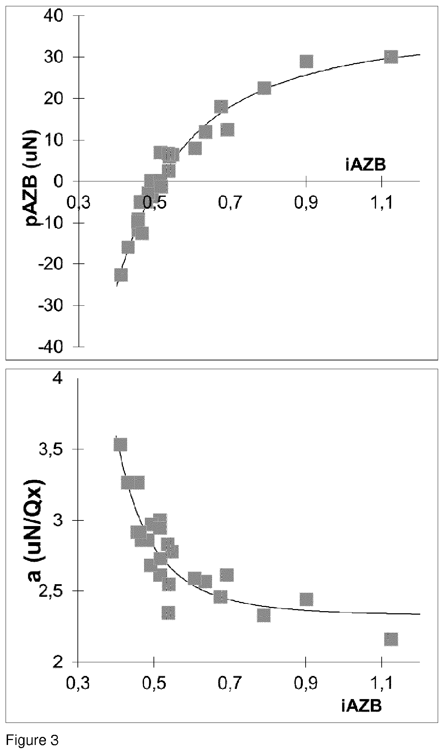

**Figure 3** : Progression asymptotique des deux principaux éléments de la méthode de calcul de dN = a x pRDT - pAZB, à savoir ; (i) pAZB, le potentiel des azotobactéries du sol à apporter de l'azote au sol (uN), et (ii) le coefficient a représentant les besoins unitaires de la culture compte tenue d'eAZB. Notons que ces valeurs de pAZB et a sont réalistes, soit de l'ordre de +/- 20 uN pour pAZB et de 2,5 à 3,5 uN/Qx pour a.

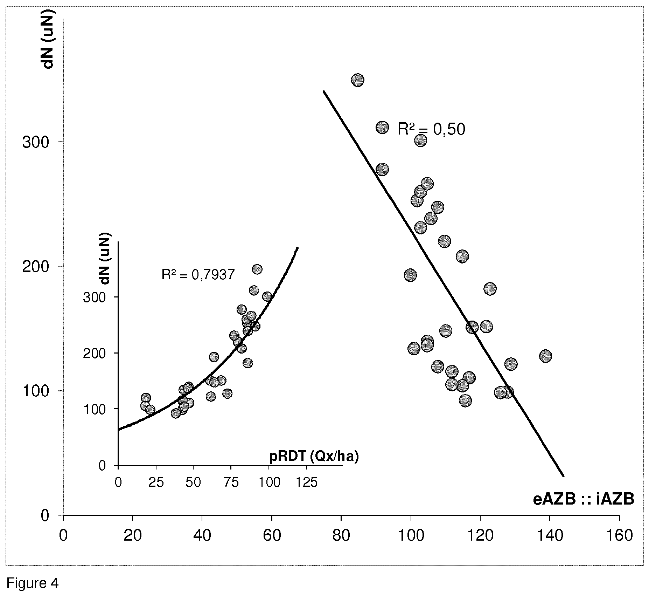

**Figure 4** : Illustration d'une des applications agronomiques de l'invention. L'établissement de la dN selon iAZB, et donc implicitement eAZB, selon le potentiel de rendement (pRDT) en blé d'hiver. En abîme, la progression de dN selon pRDT, progression exponentielle du fait d'une baisse des rendements unitaire

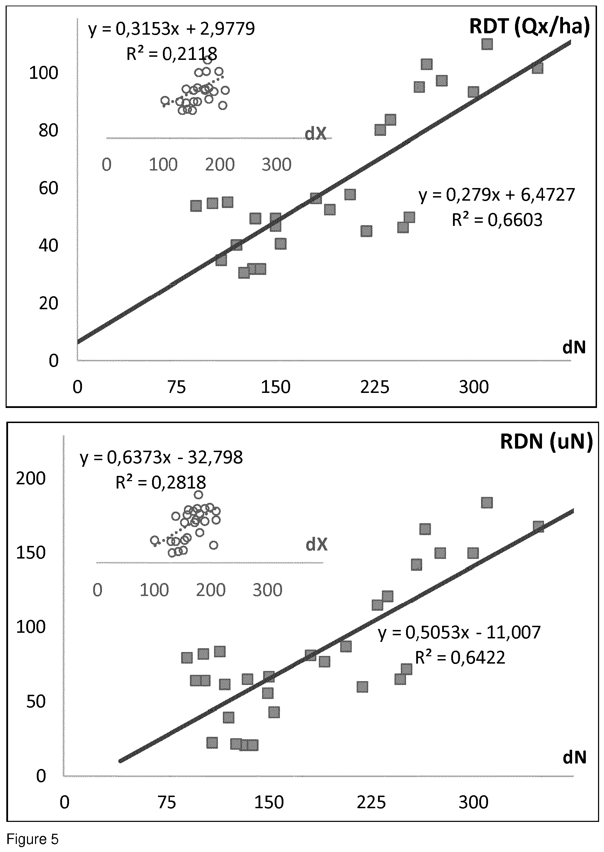

**Figure 5** : Déterminisme des dX et dN. Les coefficients de détermination $R^2$ pour les corrélations RDT et RDN par rapport à dN et dX (en abîmes) sont beaucoup plus importants avec dN qu'avec dX. Cela démontre la plus grande pertinence agronomique et environnementale de dN. Notons qu'il ne s'agit pas ici d'un simple artéfact en raison de l'existence de dN importante ce qui permet d'étendre la relation dN :: RDT(N).

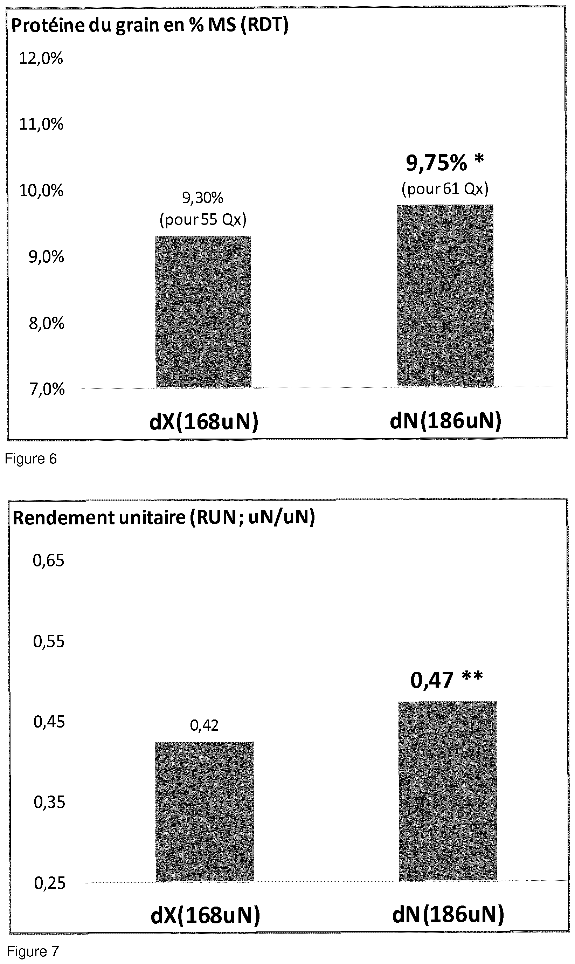

**Figure 6** : Les taux de protéine des grains de blés sont eux aussi plus élevés avec dN et cela conjointement à une augmentation des RDT (de 55 à 61 Qx/ha) du fait d'une dN plus importante que dX ; cela semble être le fait de rendements unitaires plus importants avec dN (Figure 7).

**Figure 7** : Bien qu'en moyenne supérieur d'une vingtaine d'uN (186 uN pour dN par rapport à 168 pour dX), dN ne semble pas réduire les rendements unitaires (RUN), dans l'ensemble déjà assez faibles ici, comme nous aurions pu nous y attendre. Nb. Ces RUN ont été ajustés en fonction du taux de fertilisation de manière à les rendre comparables, une augmentation de la dose d'engrais provoquant nécessairement une réduction de RUN.

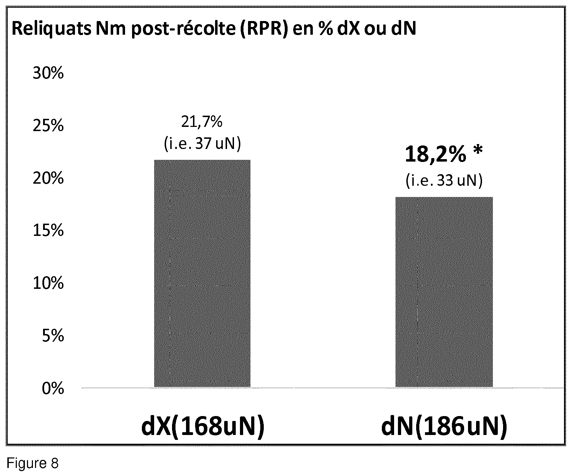

**Figure 8** : dN permettrait de réduire les reliquats post-récolte (RPR) d'$N_{minéral}$, et cela bien que dN soit ici en moyenne supérieure à dX d'une vingtaine d'uN. Il s'agit ici des RPR par rapport à la dNX ; pour info, en parenthèses les RPR en uN.

## MODE DE RÉALISATION PRÉFÉRÉ DE L'INVENTION (eAZB)

La dX, disons dorénavant dN pour marquer la différence, pourra ainsi être mieux adaptée à la fertilisation raisonnée en présence de RCS, et du coup plus apte à en optimiser eMIC, voire plus avantageusement eAZB. Expérimentalement, nous verrons comment cette synergie eAZB - dN contribuera au développement de cette nouvelle pratique qu'est la *fertilisation azotobactérienne raisonnée* (FAR). Il ne s'agit pas ici de simplement modifier (fignoler) la méthode du bilan, en ajustant par exemple le terme Fns (fixation non symbiotique de l'azote), mais bien de redéfinir dX → dN sur la base de la valeur d'eAZB escompté à partir d'une analyse multicritère quantitative. Nb. eAZB est ici un cas avantageux et particulier d'eMIC est servira d'exemple afin de démontrer la faisabilité et l'applicabilité d'un mode de réalisation l'invention.

En collaboration avec Agronutrition SAS (31390 Carbonne, France), eAZB pour divers types de RCS fut évaluée in situ sur blé et colza de 2010 à 2015. Connaissant les itinéraires techniques et les conditions pédoclimatiques, des indicateurs élémentaires d'eAZB en proximité des RCS enfouis (résidusphères) ont par la suite été déterminés et agrégés en un seul indicateur, iAZB, par pondération selon leurs valeurs propres provenant d'une analyse multivariée de type analyse par composante principale. eAZB est ici le ratio des rendements avec et sans azotobactérisation des RCS. Une fois cette eAZB évaluée, divers indicateurs élémentaires d'eAZB peuvent être conçus et par la suite été agrégés en un seul indicateur agrégé, iAZB, par normalisation (par rapport aux maxima) et pondération (selon leurs valeurs propres provenant d'analyses multivariées). Les indicateurs élémentaires utilisés ici pour la formation d'iAZB (iVAZ, iPAZ, iRES, etc. ; Tableau 1) ont été dérivés de modélisations dynamiques (Tableau 2) des variables agro - pédoclimatiques provenant pour l'essentiel de simples bulletins d'analyses de terres, stations météorologiques, voire de l'intégration de diverses bases de données (Dumanski et al. 1993).

Pour l'équation (1) soit applicable il faut normaliser les scores des indicateurs élémentaire (Si) ; à titre d'exemple au Tableau 3, la normalisation des scores de l'indicateur élémentaire iDAM (cf. Tableau 1). L'équation (1) requiert aussi un facteur de pondération (Pi) applicable au Si de chaque indicateur élémentaire. Pour faire simple, j'ai utilisé ici les valeurs propres d'un ACP (analyse par composantes principales ; Lebart et al. 2000) des données au Tableau 1 (Tableau 4). Or, cette relation eAZB - iAZB est mieux décrite par une fonction logistique avec asymptotes (Tableau 5), du moins ici en termes de $RUN_{RDN}$, le paramètre le plus accomplie du fait qu'il intègre performance agronomique *et* protéique. Comme nous pouvons le voir à la Figure 4, les valeurs de dN calculés à partir de eAZB, elle-même fonction d'iAZB, sont proportionnelles au potentiel de rendement (pRDT) ; plus cette eAZB - RUN est importante, plus les dX et dN sont faibles, ce qui rappel bien la contribution des azotobactéries au régime azoté de la culture.

**Tableau 1** : Valeurs empiriques de l'efficacité de l'azotobactérisation des RCS (eAZB) et ses indicateurs élémentaires (iAPC, iDAM, etc.) pour les susdits trente (30) essais d'azotobactéristion des RCS. eAZB est exprimée en pourcentage du rendement protéique (RDN ; kg-$N_{grain}$/ha) et unitaire (RUN ; kg-$N_{grain}$/uN) par rapport à un témoin apparié non traité. Les indicateurs élémentaires ont été déterminées selon les protocoles décrits dans les brevets listés ici à la section Sigles de définitions sur la base des données pédoclimatiques au Tableau 2. Ce sont ces valeurs qui ont été normalisées (eg. Tableau 3) de manières à obtenir Si lors du calcul de iAZB (cf. Tableaux 4, 5 et 6).

| Essai | $erAZB_{RDN}$ | $erAZB_{RUN}$ | iAPC | iDAM | iREC | iRES | iVAZ | iPAZ |
|-------|---------------|---------------|------|------|------|------|------|------|
| 1 | 105,5 | 115 | 11 | 4312 | 0,672 | -2,35 | 0,07 | 7,36E-04 |
| 2 | 122,8 | 123 | 11 | 5115 | 0,748 | -2,14 | 0,02 | 4,63E-03 |
| 3 | 99,7 | 100 | 15 | 3130 | 0,795 | -2,36 | 0,12 | 3,97E-04 |
| 4 | 113,6 | 122 | 15 | 5007 | 0,845 | -2,16 | 0,04 | 1,74E-03 |
| 5 | 104,2 | 118 | 5 | 4597 | 0,84 | -2,39 | 0,08 | 6,41E-04 |
| 6 | 117,8 | 129 | 31 | 5814 | 0,746 | -2,09 | 0,03 | 2,72E-03 |
| 7 | 96,3 | 101 | 14 | 3197 | 0,864 | -2,4 | 0,15 | 2,77E-04 |
| 8 | 95,2 | 105 | 14 | 3482 | 0,929 | -2,91 | 0,17 | 2,46E-04 |
| 9 | 104,1 | 128 | 12 | 5692 | 0,7 | -2,31 | 0,08 | 6,34E-04 |
| 10 | 127,9 | 139 | 5 | 7199 | 0,722 | -2,33 | 0,02 | 7,96E-03 |
| 11 | 97,8 | 105 | 4 | 3482 | 1,036 | -2,73 | 0,14 | 3,24E-04 |
| 12 | 109 | 118 | 10 | 4597 | 0,704 | -2,78 | 0,06 | 1,07E-03 |
| 13 | 113 | 117 | 6 | 4500 | 0,749 | -2,1 | 0,04 | 1,63E-03 |
| 14 | 100 | 112 | 15 | 4044 | 0,895 | -2,73 | 0,11 | 4,10E-04 |
| 15 | 115 | 115 | 15 | 4312 | 0,728 | -2,55 | 0,04 | 2,02E-03 |

(suite)

| Essai | erAZB$_{RDN}$ | erAZB$_{RUN}$ | iAPC | iDAM | iREC | iRES | iVAZ | iPAZ |
|---|---|---|---|---|---|---|---|---|
| 16 | 111 | 116 | 15 | 4405 | 0,767 | -2,76 | 0,05 | 1,32E-03 |
| 17 | 100 | 102 | 19 | 3266 | 0,895 | -2,38 | 0,11 | 4,10E-04 |
| 18 | 104 | 108 | 19 | 3713 | 0,845 | -3,06 | 0,08 | 6,27E-04 |
| 19 | 99 | 110 | 19 | 3875 | 0,909 | -2,56 | 0,12 | 3,69E-04 |
| 20 | 100 | 108 | 19 | 3713 | 0,895 | -3,12 | 0,11 | 4,10E-04 |
| 21 | 109 | 112 | 19 | 4044 | 0,788 | -2,25 | 0,06 | 1,07E-03 |
| 22 | 121 | 126 | 19 | 5454 | 0,675 | -2,9 | 0,03 | 3,82E-03 |
| 23 | 85,4 | 85 | 6 | 2272 | 1,131 | -2,96 | 0,4 | 8,68E-05 |
| 24 | 92,4 | 103 | 6 | 3337 | 1,007 | -3,04 | 0,21 | 1,83E-04 |
| 25 | 92,1 | 92 | 12 | 2638 | 1,011 | -2,25 | 0,22 | 1,77E-04 |
| 26 | 91,5 | 103 | 12 | 3337 | 1,021 | -2,4 | 0,23 | 1,66E-04 |
| 27 | 91 | 92 | 6 | 2638 | 1,03 | -2,86 | 0,24 | 1,57E-04 |
| 28 | 92,9 | 105 | 6 | 3482 | 0,999 | -2,21 | 0,2 | 1,93E-04 |
| 29 | 94,6 | 103 | 5 | 3337 | 0,972 | -2,61 | 0,18 | 2,31E-04 |
| 30 | 107 | 106 | 5 | 3558 | 0,81 | -2,68 | 0,07 | 8,63E-04 |

**Tableau 2** : Sommaire statistiques des données pédoclimatiques pour les trente (30) essais agronomiques récoltés de 2011 à 2015 pour lesquelles existent des valeurs d'efficacité de l'azotobactérisation des RCS (résidus de culture au sol) par rapport à un témoin non traité (TNT) et qui servirent au calcul des indicateurs élémentaires, iAZB™, d'eAZB utilisés ici. (RCS - type de résidus de culture au sol ; APC - agro-pédo-climat au sens de EP13366005.0 ; rCNsol - ratio carbone sur azote de la matière organique du sol ; N et Corg - azote et carbone du sol ; RAT - résidus automnaux d'azote minéral ; RSH-résidus sortie hiver d'azote minéral, ammoniacal (NH4) et nitrique (NO3) ; rNN - ratio N-NH4 sur N-NO3 d'un échantillon ou prélèvement de terre ; dpH = pHKCl - pHeau ; N et Prcs - teneurs en N et P des RCS ; rCN, CP et NPrcs - ratios C sur N, C sur P et N sur P des RCS.

| | APC | rCNsol | Norg | Corg | Argile | RAT | RSH-NH4 | RSH-NO3 | RSH | rNN | pHeau | pHKCl | dpH | RCS | Nrcs | Prcs | rCNrcs | rCPrcs | rNPrcs |
|---|---|---|---|---|---|---|---|---|---|---|---|---|---|---|---|---|---|---|---|
| **Moyennes** | | | | | | | | | | | | | | | | | | | |
| BTH (n = 14) | 14 | 9 | 0,130 | 1,19 | 30,6 | 32,1 | 2,93 | 25,1 | 28,1 | 0,108 | 7,7 | 6,76 | 0,91 | 9 | 2,14 | 0,065 | 90 | 3095 | 34,4 |
| CLZ (n = 6) | 11 | 10 | 0,176 | 1,76 | 23,0 | 29,2 | 5,50 | 44,5 | 50,0 | 0,117 | 6,7 | 5,66 | 1,04 | 6 | 1,02 | 0,070 | 45 | 657 | 14,6 |
| TSL(n = 3) | 14 | 10 | 0,175 | 1,75 | 30,0 | 17,0 | 3,93 | 32,6 | 36,6 | 0,118 | 6,7 | 5,66 | 1,08 | 6 | 1,02 | 0,070 | 45 | 657 | 14,6 |
| ZEA (n = 7) | 9 | 10 | 0,142 | 1,42 | 19,7 | 27,5 | 3,45 | 27,0 | 30,5 | 0,135 | 7,3 | 6,45 | 0,87 | 12 | 1,40 | 0,125 | 60 | 670 | 11,2 |
| **Écartypes** | | | | | | | | | | | | | | | | | | | |
| BTH | 5 | 1 | 0,025 | 0,33 | 9,3 | 9,1 | 2,55 | 16,1 | 18,2 | 0,060 | 1,2 | 1,31 | 0,33 | 0 | 0,00 | 0,017 | 0 | 447 | 5,0 |
| CLZ | 4 | 0 | 0,058 | 0,58 | 7,0 | 12,1 | 1,26 | 19,2 | 20,5 | 0,022 | 0,2 | 0,50 | 0,30 | 0 | 0,00 | 0,000 | 0 | 0 | 0,0 |
| TSL | 14 | 0 | 0,108 | 1,08 | 26,0 | 3,5 | 1,26 | 9,6 | 8,4 | 0,071 | 0,2 | 0,41 | 0,47 | 0 | 0,00 | 0,000 | 0 | 0 | 0,0 |
| ZEA | 4 | 0 | 0,025 | 0,25 | 8,2 | 12,3 | 3,41 | 20,3 | 22,5 | 0,065 | 0,9 | 0,83 | 0,21 | 0 | 0,00 | 0,000 | 0 | 0 | 0,0 |

EP 3 335 536 B1

**Tableau 3** : Exemple de normalisation des scores indicateurs élémentaire (Si) sur une base de 1,00 = valeur maximale. Cette transformation exemplaire ici pour l'indicateur iDAM s'effectue exactement de la même manière pour les six autres indicateurs élémentaires (Tableau 1). Voir à la Figure 1 les graphes des fonctions décrivant les relations entre ces valeurs de Si et d'eAZB.

| Essai | iDAM | iDAM (Si) | $eAZB_{RDN}$ | $eAZB_{RUN}$ |
|---|---|---|---|---|
| 1 | 4312 | 0,60 | 106 | 115 |
| 2 | 5115 | 0,71 | 123 | 123 |
| 3 | 3130 | 0,43 | 100 | 100 |
| 4 | 5007 | 0,70 | 114 | 122 |
| 5 | 4597 | 0,64 | 104 | 118 |
| 6 | 5814 | 0,81 | 118 | 129 |
| 7 | 3197 | 0,44 | 96 | 101 |
| 8 | 3482 | 0,48 | 95 | 105 |
| 9 | 5692 | 0,79 | 104 | 128 |
| 10 | 7199 | 1,00 | 128 | 139 |
| 11 | 3482 | 0,48 | 98 | 105 |
| 12 | 4597 | 0,64 | 109 | 118 |
| 13 | 4500 | 0,63 | 113 | 117 |
| 14 | 4044 | 0,56 | 100 | 112 |
| 15 | 4312 | 0,60 | 115 | 115 |
| 16 | 4405 | 0,61 | 111 | 116 |
| 17 | 3266 | 0,45 | 100 | 102 |
| 18 | 3713 | 0,52 | 104 | 108 |
| 19 | 3875 | 0,54 | 99 | 110 |
| 20 | 3713 | 0,52 | 100 | 108 |
| 21 | 4044 | 0,56 | 109 | 112 |
| 22 | 5454 | 0,76 | 121 | 126 |
| 23 | 2272 | 0,32 | 85 | 85 |
| 24 | 3337 | 0,46 | 92 | 103 |
| 25 | 2638 | 0,37 | 92 | 92 |
| 26 | 3337 | 0,46 | 92 | 103 |
| 27 | 2638 | 0,37 | 91 | 92 |
| 28 | 3482 | 0,48 | 93 | 105 |
| 29 | 3337 | 0,46 | 95 | 103 |
| 30 | 3558 | 0,49 | 107 | 106 |
| | **Valeur Maxi** **7199** | | | |

**Tableau 4** : Valeurs propres (F1 et 2) issue d'analyses par composantes principales (ACP) des données au Tableau 1 utilisées ici pour la pondération (Pi) des scores normalisés des différents indicateurs élémentaire normalisés (Si ; Tableau 6). La pondération est l'étape au cours de laquelle on attribue des poids aux indicateurs que l'on veut agréger (Lairez et al. 2015 ; pp. 123-124). On retient que la première composante principale, F1 à titre de Pi, le 2/3 environs de la variabilité étant ici appréciable par F1.

| | ----------$eAZB_{RDN}$---------- | | --------- $eAZB_{RUN}$--------- | |
|---|---|---|---|---|
| | F1 (Pi) | F2 | F1 (Pi) | F2 |
| eAZB | **0,854** | 0,001 | **0,835** | 0,010 |
| iAPC | 0,094 | **0,552** | 0,100 | **0,458** |
| iDAM | **0,953** | 0,020 | **0,969** | 0,011 |
| iREC | **0,899** | 0,011 | **0,962** | 0,000 |
| iRES | 0,059 | **0,425** | 0,036 | **0,567** |

(suite)

| | ---------- eAZB$_{RDN}$ ---------- | | --------- eAZB$_{RUN}$ --------- | |
|---|---|---|---|---|
| iVAZ | **0,622** | 0,006 | **0,782** | 0,011 |
| iPAZ | **0,628** | 0,050 | **0,741** | 0,089 |

**Tableau** 5 : Paramètres de la régression non linéaire (Logit) de type eAZB = pr1 +(pr4-pr1)/(1+(iAZB/pr3)^pr2) permettant de mieux prédire l'eAZB en terme de RUN (eAZB - RUN) à partir de iAZB par rapport à une simple régression logarithmique (Ln ; Figure 2) ; cette amélioration n'est plus très appréciable pour eAZB - RDN. Pour comparaison, voir %R2 pour ces deux régressions de eAZB sur iAZB.

| *Paramètre et* | *eAZB ~ RDN* | *eAZB ~ RUN* |
|---|---|---|
| **pr1** | 130,086 | 135,919 |
| **pr2** | 3,946 | 3,918 |
| **pr3** | 1,756 | 0,879 |
| **pr4** | 56,382 | -1303,180 |
| **%R$^2$ (Logit)** | 53,6 | 78,4 |
| **% R$^2$ (Ln)** | 54,1 | 59,7 |

**Tableau 6** : Transformation (normalisation sur une base de 1,00 = valeurs maximales) des scores des indicateurs élémentaires (Si) pour les trente (30) essais agronomique (Tableau 7) via les fonctions d'attribution des scores (Figure 1) et leurs pondération (Pi) selon leurs valeurs propres correspondantes issue de l'analyse par composantes principales (ACP ; Tableau 4). La somme des valeurs Pi x Si est ici définie par iAZB. Nb. Il est ici question d'eAZB$_{RUN}$ ; exactement la même approche est applicable avec eAZB$_{RDN}$, sauf que les valeurs de Pi ne sont pas les mêmes (Tableau 4).

| iAZB | eAZB$_{RDN}$ | eAZB$_{RUN}$ | Pi x Si | | | | | | score (Si) via FAS | | | | | |
| | | | iAPC | iDAM | iREC | iRES | iVAZ | iPAZ | iAPC | iDAM | iREC | iRES | iVAZ | iPAZ |
|---|---|---|---|---|---|---|---|---|---|---|---|---|---|---|
| 2,42 | 106 | 115 | 0,035 | 0,6 | 0,777 | 0,026 | 0,147 | 0,069 | 0,35 | 0,62 | 0,81 | 0,74 | 0,19 | 0,09 |
| 3,558 | 123 | 123 | 0,035 | 0,671 | 0,764 | 0,002 | 0,557 | 0,431 | 0,35 | 0,9 | 0,69 | 0,79 | 0,06 | 0,58 |
| 2,418 | 100 | 100 | 0,047 | 0,829 | 0,836 | 0,01 | 0,048 | 0,037 | 0,47 | 0,55 | 0,86 | 0,87 | 0,29 | 0,05 |
| 2,762 | 114 | 122 | 0,047 | 0,726 | 0,79 | 0,004 | 0,21 | 0,162 | 0,47 | 0,74 | 0,75 | 0,82 | 0,1 | 0,22 |
| 2,507 | 104 | 118 | 0,017 | 0,792 | 0,937 | 0,007 | 0,077 | 0,06 | 0,17 | 0,6 | 0,82 | 0,97 | 0,21 | 0,08 |
| 3,227 | 118 | 129 | 0,1 | 0,7 | 0,907 | 0,003 | 0,327 | 0,253 | 1 | 0,81 | 0,72 | 0,94 | 0,08 | 0,34 |
| 2,293 | 96 | 101 | 0,044 | 0,858 | 0,823 | 0,014 | 0,033 | 0,026 | 0,44 | 0,51 | 0,89 | 0,86 | 0,39 | 0,03 |
| 2,425 | 95 | 105 | 0,044 | 0,868 | 0,962 | 0,015 | 0,03 | 0,023 | 0,44 | 0,5 | 0,9 | 1 | 0,42 | 0,03 |
| 2,342 | 104 | 128 | 0,038 | 0,793 | 0,797 | 0,007 | 0,076 | 0,059 | 0,38 | 0,6 | 0,82 | 0,83 | 0,21 | 0,08 |
| 4,18 | 128 | 139 | 0,016 | 0,644 | 0,671 | 0,001 | 0,958 | 0,741 | 0,16 | 1 | 0,67 | 0,7 | 0,04 | 1 |
| 2,191 | 98 | 105 | 0,014 | 0,845 | 0,705 | 0,012 | 0,039 | 0,03 | 0,14 | 0,53 | 0,87 | 0,73 | 0,34 | 0,04 |
| 2,58 | 109 | 118 | 0,033 | 0,757 | 0,905 | 0,005 | 0,128 | 0,099 | 0,33 | 0,67 | 0,78 | 0,94 | 0,14 | 0,13 |
| 2,577 | 113 | 117 | 0,019 | 0,73 | 0,706 | 0,004 | 0,197 | 0,152 | 0,19 | 0,73 | 0,75 | 0,73 | 0,11 | 0,21 |
| 2,406 | 100 | 112 | 0,05 | 0,826 | 0,856 | 0,01 | 0,049 | 0,038 | 0,5 | 0,55 | 0,85 | 0,89 | 0,29 | 0,05 |
| 2,709 | 115 | 115 | 0,05 | 0,717 | 0,695 | 0,003 | 0,243 | 0,188 | 0,5 | 0,76 | 0,74 | 0,72 | 0,09 | 0,25 |
| 2,53 | 111 | 116 | 0,05 | 0,743 | 0,712 | 0,004 | 0,159 | 0,123 | 0,5 | 0,7 | 0,77 | 0,74 | 0,13 | 0,17 |
| 2,22 | 100 | 102 | 0,06 | 0,826 | 0,664 | 0,01 | 0,049 | 0,038 | 0,6 | 0,55 | 0,85 | 0,69 | 0,29 | 0,05 |
| 2,354 | 104 | 108 | 0,06 | 0,794 | 0,734 | 0,007 | 0,076 | 0,058 | 0,6 | 0,6 | 0,82 | 0,76 | 0,21 | 0,08 |
| 2,351 | 99 | 110 | 0,06 | 0,834 | 0,806 | 0,011 | 0,044 | 0,034 | 0,6 | 0,54 | 0,86 | 0,84 | 0,31 | 0,05 |
| 2,461 | 100 | 108 | 0,06 | 0,826 | 0,904 | 0,01 | 0,049 | 0,038 | 0,6 | 0,55 | 0,85 | 0,94 | 0,29 | 0,05 |
| 2,514 | 109 | 112 | 0,06 | 0,757 | 0,763 | 0,005 | 0,128 | 0,099 | 0,6 | 0,67 | 0,78 | 0,79 | 0,14 | 0,13 |
| 3,239 | 121 | 126 | 0,06 | 0,682 | 0,744 | 0,002 | 0,46 | 0,356 | 0,6 | 0,86 | 0,7 | 0,77 | 0,06 | 0,48 |
| 2,215 | 85 | 85 | 0,02 | 0,969 | 0,775 | 0,036 | 0,01 | 0,008 | 0,19 | 0,4 | 1 | 0,81 | 1 | 0,01 |
| 2,323 | 92 | 103 | 0,02 | 0,895 | 0,89 | 0,019 | 0,022 | 0,017 | 0,19 | 0,47 | 0,92 | 0,93 | 0,54 | 0,02 |
| 2,272 | 92 | 92 | 0,039 | 0,898 | 0,77 | 0,02 | 0,021 | 0,016 | 0,39 | 0,47 | 0,93 | 0,8 | 0,55 | 0,02 |
| 2,136 | 92 | 103 | 0,039 | 0,904 | 0,635 | 0,021 | 0,02 | 0,015 | 0,39 | 0,46 | 0,93 | 0,66 | 0,58 | 0,02 |
| 2,118 | 91 | 92 | 0,02 | 0,909 | 0,687 | 0,021 | 0,019 | 0,015 | 0,19 | 0,45 | 0,94 | 0,71 | 0,6 | 0,02 |
| 2,362 | 93 | 105 | 0,02 | 0,89 | 0,927 | 0,018 | 0,023 | 0,018 | 0,19 | 0,47 | 0,92 | 0,96 | 0,51 | 0,02 |
| 2,15 | 95 | 103 | 0,015 | 0,874 | 0,717 | 0,016 | 0,028 | 0,022 | 0,15 | 0,49 | 0,9 | 0,75 | 0,44 | 0,03 |

(suite)

| iAZB | Pi x Si | | | | | | | | score (Si) via FAS | | | | | |
|---|---|---|---|---|---|---|---|---|---|---|---|---|---|---|
| | eAZB$_{RDN}$ | eAZB$_{RUN}$ | iAPC | iDAM | iREC | iRES | iVAZ | iPAZ | iAPC | iDAM | iREC | iRES | iVAZ | iPAZ |
| 2,544 | 107 | **106** | 0,015 | 0,772 | 0,93 | 0,006 | 0,104 | 0,08 | 0,15 | 0,64 | 0,8 | 0,97 | 0,17 | 0,11 |

Tableau 7 : Paramétrage du calcul de dN pour une culture de blé tendre d'hiver par rapport à dX sur la base du potentiel azotobactérien des RCS (pAZB) et du rendement du témoin non traité (TNT). La dX rapportée ici par l'expérimentateur a été calculée selon la méthode du bilan Comifer.

| Essai | pRDT (TNT) | $b_{Arvalis}$ | a | pAZB | dX | dN |
|---|---|---|---|---|---|---|
| 1 | 57,4 | 3 | 2,61 | 6,3 | 174 | 150 |
| 2 | 57,4 | 3 | 2,44 | 22,3 | 154 | 124 |
| 3 | 60,1 | 3 | 3,00 | -0,4 | 210 | 180 |
| 4 | 60,1 | 3 | 2,46 | 14,4 | 190 | 133 |
| 5 | 58,3 | 3 | 2,54 | 4,4 | 102 | 135 |
| 6 | 60,2 | 3 | 2,33 | 18,1 | 130 | 121 |
| 7 | 43,3 | 3 | 2,97 | -3,5 | 143 | 137 |
| 8 | 43,3 | 3 | 2,86 | -4,5 | 133 | 133 |
| 9 | 40 | 3 | 2,34 | 3,2 | 56 | 91 |
| 10 | 59,6 | 3 | 2,16 | 28,4 | 152 | 112 |
| 11 | 51,34 | 3 | 2,86 | -2,0 | 173 | 131 |
| 12 | 51,5 | | | | | |
| 13 | 42,9 | 3 | 2,56 | 9,8 | 205 | 99 |
| 14 | 42,96 | 3 | 2,68 | 0,0 | 180 | 118 |
| 15 | 42,72 | 3 | 2,61 | 11,5 | 160 | 103 |
| 16 | 36,95 | 3 | 2,59 | 8,7 | 140 | 105 |
| 17 | 85,78 | 3 | 2,94 | 0,0 | 180 | 235 |
| 18 | 85,22 | 3 | 2,78 | 6,2 | 160 | 216 |
| 19 | 76,83 | 3 | 2,73 | -1,6 | 140 | 220 |
| 20 | | | | | | |
| 21 | | | | | | |
| 22 | | | | | | |
| 23 | 97,6 | 3 | 3,53 | -30,8 | 177 | 348 |
| 24 | 97,6 | 3 | 2,91 | -13,2 | 177 | 246 |
| 25 | 72 | 3 | 3,26 | -12,9 | 162 | 257 |
| 26 | 72 | 3 | 2,91 | -13,9 | 162 | 232 |
| 27 | 92,2 | 3 | 3,26 | -18,3 | 179 | 320 |
| 28 | 92,2 | 3 | 2,86 | -14,1 | 179 | 278 |
| 29 | 79,7 | 3 | 2,91 | -9,7 | 198 | 257 |
| 30 | 79,7 | 3 | 2,83 | 11,1 | 198 | 229 |
| MOYENNE -> | 64 | 3.00 | 2,77 | 0,74 | 162 | 181 |

## APPLICATIONS BIOINDUSTRIELLES ET AGRONOMIQUES (eAZB → dN)

Les données agronomiques aux Tableaux 1 et 2 servirent à une série de modélisations de tels systèmes de culture à l'aide de l'outil Stics™ (Brisson et al. 2008). En ce sens, à la Figure 5 nous voyons que le déterminisme (i.e. $R^2$) des dX et dN. Les coefficients de détermination $R^2$ pour les corrélations RDT et RDN par rapport à dN et dX (en abîmes) sont beaucoup plus importants avec dN qu'avec dX. Cela démontre la plus grande pertinence agronomique et environnementale de dN. Notons qu'il ne s'agit pas ici d'un simple artéfact graphique en raison de l'existence de dN plus importantes ce qui permettrait simplement d'étendre la relation dN :: RDT(N). L'utilisation des dN par rapport aux simples dX permet aussi d'augmenter significativement (t.Test ; XLStats™) les taux de protéines des grains (Figure 6), ainsi que les rendements unitaires (RUN ; uN/uN) ajustés selon les valeurs de dN et dX foncièrement différentes (Figure 7).

Enfin, la Figure 8 révèle que dN permettra aussi de réduire les *reliquats post-récolte* (RPR) d'$N_{minéral}$, et cela bien que dN soit ici en moyenne supérieure d'environ 20 uN à dX. Notons qu'il s'agit des RPR par rapport à dX ou dN (en parenthèses les RPR en uN sans cet ajustement). Pour terminer, notons qu'il existe une synergie entre les engrais à libération contrôlée, ou ELC (CRF ; *controlled release fertilizer),* ou encore des engrais à libération, ou dissolution, lente (EDL, ou en anglais SRF ; *slow release fertilizers),* et la FAR. En effet, l'activité des AZB sur résidus de culture « surimmobilise » les RAT d'Nm et les relargue dès la sortie d'hiver ; *idem* pour l'azote d'origine diazotrophe. C'est vraisemblablement pourquoi ces AZB profitent d'un report d'une partie de N1 (le premier apport d'engrais N) vers N2, 3 ou 4. Ledit report N1 → N234 pourrait très bien être effectué à l'aide de CRF

***Références, bibliographie et brevets pertinents***

Andrews, Susan S. Jeffrey P. Mitchell, Roberto Mancinelli, Douglas L. Karlen, Timothy K. Hartz, William R. Horwath, G. Stuart Pettygrove, Kate M. Scow et Daniel S. Munk. 2002. On-Farm Assessment of Soil Quality in California's Central Valley. Agron. J. 94:12-23 (2002)

Andrews, SS. DL. Karlen et CA. Cambardella. 2004. The Soil Management Assessment Framework : A Quantitative Soil Quality Evaluation Method. Soil Sci. Soc. Am. J. 68:1945-1962 (2004).

Arvalis 2014. « b variétaux » : besoin en azote au quintal - Perspectives. Institut du végétal / COMIFER.

Arvalis 2016. Gérer la fertilisation du blé sans la méthode du bilan. Réussir Grandes Cultures no. 307 (novembre 2016), pp. 32-34 (Auteure : Valérie Noël)

Bastida, F, A. Zsolnay, T. Hernández et C. García. 2008. Past, présent and future of soit quality indicateurs: A biological perspective. Geoderma 147 (2008) 159-171

Bissonnais, Le Y et Souder, Le C. 1995. Mesurer la stabilité structurale des sols pour évaluer leur sensibilité à la battance et à l'érosion. Étude et Gestion des Sols 2(1) : 43-56

Brisson, N, M. Launay, B. Mary et N. Beaudoin (eds). 2008. Conceptual basis, formalisations and parameterization of the STICS crop model. Editions Quae, c/o Inra RD 10, 78026 Versailles Cedex.

Boukherroub, Tasseda, Alain Guinet, Julien Fondrevelle. Méthode d'aide à la décision multi-critières pour l'internalisation/externalisation "durable". 9th International Conférence on Modeling, Optimization & SIMulation, Jun 2012, Bordeaux, France. 2012. <hal-00728639>

Cinelli, Marco, Stuart R Coles et Kerry Kirwan. 2014. Analysis of the potentiels of multi criteria décision analysis methods to conduct sustainability assessment. Ecological Indicators 46 (2014) 138-148

Claude, P.-P., MacKeruie, A. F, Coulman, B. E. et Mehuys, G. R. 1993. Effet du labour printanier et des cultures intercalaires de trèfle rouge sur le rendement du maïs-grain. Can. J. Plant Sci. 73: 37-46. (http://www.nrcresearchpress.com/doi/pdfplus/10.4141/cjps93-006)

Claude, P-P. et M. Giroux. 2006. Effet des engrais organo-minéraux inoculés (EOMI) sur la croissance des plants de maïs-grain, les rendements, les prélèvements des éléments nutritifs et la qualité des grains. Agrosol. 17: 51-64. (https://www.irda.qc.ca/assets/documents/Publications/documents/claude-giroux-2006 article effet eomi mais.pdf)

Claude, P-P. et L. Fillion. 2004. Effet de l'apport d'un inoculum bactérien aux résidus de culture de maïs-grain au sol sur le rendement et la qualité de blés d'hiver panifiables en France. Agrosol 15: 23-29. (http://collections.banq.qc.ca/ark:/52327/bs604022)

COMIFER 2013. Calcul de la fertilisation azotée : Guide méthodologique pour l'établissement des prescriptions locales Édition 2013 - Cultures annuelles et prairies. Brochure éditée par le Le Diamant A 92909 Paris La Défense Cedex (Dépôt légal : Avril 2011 ISBN 978-2-910393-09-0)

Dumanski, J., W.W. Pettapiece, D.F. Acton et P-P. Claude. 1993. Application of agro-ecological concepts and hierarchy theory in the design of databases for spatial and temporal characterisation of land and soil. Geoderma 60 : 343-358. (http://www.sciencedirect.com/science/article/pii/001670619390Q35J)

Felske, A, A.D.L. Akkermans. 1998. Spatial Homogeneity of Abundant Bacterial 16S rRNA Molécules in Grassland Soils. Microb Ecol (1998) 36:31-36

Glover, JD, JP Reganold et PK Andrews. 2000. Systematic method for rating soit quality of conventional, organic, and integrated apple orchards in Washington State. Agriculture, Ecosystems and Environment 80 : 29-45

Horwath, William R., G. Stuart Pettygrove, Kate M. Scow et Daniel S. Munk. 2002 bis. On-Farm Assessment of Soil Quality in California's Central Valley. Agron. J. 94:12-23 (2002).

Justes, E, B Mary, JM Meynard, JM Machet et L Thelier-Huche. 1994. Determination of a critical nitrogen dilution curve for winter wheat crops. Ann. Bot. 74 : 397-407

Knoepp, JD., DC. Coleman, DA. Crossley Jr, et JS. Clark. 2000. Biological indicateurs of soil quality: an ecosystem case study of their use. Forest Ecology and Management 138 (2000) 357-368

Lairez, J, P Feschet, J Aubin, Ch Boskstaller et I Bouvarel. 2015. Agriculture et développement durable : guide pour l'évaluation multicritère. Eds. Quae, 78026 Versailles Cedex

Lebart, L, A Morineau et M Piron. 2000. Statistiques exploratoire multidimensionnelles. Eds. Dunod, Paris.

Morvan, T., L. Beff et Y. Lambert. 2015. Minéralisation de l'azote des sols (Ouest) : résultats du projet "Mh". Rencontres de la fertilisation raisonnée COMIFER 2015, 18 & 19 novembre 2015 - Lyon 12èmes

Mulvaney, RL, S. A. Khan, and T. R. Ellsworth. 2009. Synthetic Nitrogen Fertilizers Deplete Soil Nitrogen: A Global Dilemma for Sustainable Cereal Production. J. Environ. Qual. 38:2295-2314 (2009). doi:10.2134/jeq2008.0527

Ravier, Clémence, Jean-Marc Meynard, Jean-Pierre Cohan et Philippe Gate et Marie-Hélène Jeuffroy. 2017. Early nitrogen deficiencies favor high yield, grain protein content and N use efficiency in wheat. European Journal of Agronomy 89 (2017) 16-24

Ravier, Clémence, Marie-Hélène Jeuffroy et Jean-Marc Meynard. 2016. Mismatch between a science-based decision tool and its use: The case of the balance-sheet method for nitrogen fertilization in France. NJAS - Wageningen Journal of Life Sciences xxx (2016) xxx-xxx (in press)

Ravier, Clémence, Marie-Hélène Jeuffroy et Jean-Marc Meynard. 2015. Les travaux des GREN, révélateurs de controverses autour de la méthode du bilan. Rencontres de la fertilisation raisonnée COMIFER 2015, 18 & 19 novembre 2015 - Lyon 12èmes

Saaty, Thomas L. 2008. Decision making with the analytic hierarchy process. Int. J. Services Sciences, Vol. 1, No. 1, 2008 83

Schloter, M, O. Dilly et JC Muncha. 2003. Indicators for evaluating soil quality. Agriculture, Ecosystems and Environment 98 (2003) 255-262

**Revendications**

1. Méthode de fertilisation-N raisonnée pour grandes cultures agronomiques comportant la formation, la calibration, la préconisation et l'épandage au champs de doses d'engrais N prévisionnelles comprenant les étapes suivantes ;

   • la détermination de *n indicateurs élémentaires* de l'*efficacité microbienne* (*eM/C*) de microorganismes phytogènes du système sol-plant, y compris ceux réintroduits par inoculation, à augmenter les rendements agronomiques (RDT), protéiques (RDN) ou unitaires (RUN) de la culture agronomique par rapport à un témoin,
   • l'agrégation de ces n indicateurs (i) élémentaires d'eMIC, après leurs transformations en autant de scores normalisés et pondérés, en un *indicateur agrégé* (*iMIC*) provenant donc de l'agrégation de ces n indicateurs élémentaires obtenus des données pédoclimatiques de la couche arable,
   • mise en relation d'eMIC avec iMIC,
   • la calibration de ces doses d'engrais-N (*dN*) est fonction d'eMIC ainsi déduites empiriquement d'iMIC,
   • pesée, préconisation et épandage des engrais N correspondant à dN et dans laquelle;

   l'agrégation des indicateurs élémentaires en indicateurs agrégés, par exemple ici *iMIC,* ce fait somme suit ;

$$iMIC = \sum(Si \times Pi) \text{ pour les } i = 1, 2, 3 \ldots \rightarrow \text{nombre d'indicateurs } (n)$$

*Si* étant ici le score normalisé (base 100 ou 1,00) de l'indicateur élémentaire affectant eMIC, et *Pi* le poids (0,00 à 1,00) attribué à ce score, pMIC est ici en sorte la contribution (fourniture) en N des microorganismes phytogènes du sol, y compris ceux réintroduits par l'inoculation des RCS, et dans laquelle; la préconisation et l'application pondérale in situ précises de la dose d'engrais N (dN) en fonction de l'indicateur agrégé, iMIC, lui-même obtenu par agrégation des indicateurs élémentaires de la capacité des microorganismes phytogènes du sol, y compris ceux réintroduits par inoculation au système sol - plant, ou encore stimulés in situ par application de molécules signalétiques ou bio-stimulantes, à augmenter RDN et RUN étant calculé comme suite ;

$$dN = a \times pRDT - pMIC$$

sachant que ;

- $a = b_{ARVALIS} / eMIC_{RUN}$
- $pMIC = pRDN - pRDN/eMIC_{RDN}$

et que pRDT et pRDN sont les objectifs, ou potentiels, de rendements agronomiques ($Qx_{grain}$/ha) et protéiques (kg-$N_{grain}$/ha), $eMIC_{RDN}$ et $eMIC_{RUN}$ les valeurs d'eMIC en termes de RDN et RUN (rendements unitaires ; kg-$N_{grain}$/unité d'N fertilisant), respectivement et par rapport à un témoin non traité, et que a est le coefficient b dit d'Arvalis™ ($b_{ARVALIS}$), alias « besoin unitaire » (i.e. unités d'N par $Qx_{grain}$), divisée par $eMIC_{RUN}$, pMIC étant ici en sorte la contribution (fourniture) en N des microorganismes résidusphériques phytogènes, y compris ceux réintroduits par l'inoculation, la formation de la dose d'engrais N et/ou P à partir de matières fertilisantes connues, sa préconisation sous forme d'apports fractionnés, et son épandage au champ étant eux pour le reste effectués de manières conventionnelles.

2. Méthode de fertilisation-N raisonnée pour grandes cultures agronomiques selon la revendication précédente **caractérisée en ce que** les indicateurs élémentaires d'eMIC agrégés en indicateurs agrégés ou composites obtenus par échantillonnage de la couche arables de parcelles agricoles sont choisi parmi un groupe comprenant ;

- iAPC, i.e. un indicateur élémentaire du niveau de spécificité agropédoclimatique des biomasses azotobactérienne d'un échantillon de terre provenant d'une à une parcelle agricole ;
- iRES, i.e. un indicateur élémentaire du niveau de résilience de la microflore bactérienne d'un sol en termes de diversité, d'activité et/ou d'abondance confrontée à des variations de pH du sol ;
- iREC, i.e. un indicateur élémentaire de l'efficience carbonée (eC) de la microflore tellurique, notamment azotobactérienne, en proximité des RCS ;
- iDAM, i.e. un indicateur élémentaire de l'état diazotrophe du sol comportant qu'un relevé des RAT et un indicateur d'activité diazotrophe des bactéries du sol.
- iVAZ, i.e. un indicateur élémentaire de l'activité des azotobactéries du sol intégrant la quantité de RCS et leurs teneurs en N, ainsi que les valeurs RVN = [ RCS / Nrcs ] et rMS = [ RCS/ RAC ] ;
- iPAZ, i.e. un indicateur élémentaire de la quantité d'engrais P nécessaire à la flore azotobactérienne résidusphérique pour fonctionner de manière optimale en termes agronomique.

3. Méthode de fertilisation N raisonnée pour grandes cultures agronomiques selon une quelconque des revendications précédentes **caractérisée en ce que** la transformation les n différents indicateurs élémentaires (i) d'eMIC en autant de scores normalisés (Si) ce fait à l'aide de *n* fonctions d'attribution de scores (alias FAS, ou fonction d'utilités) décrivant ces indicateurs élémentaires corrélés avec l'efficacité microbienne (eMIC) de microorganismes phytogènes du système sol-plant, y compris ceux réintroduits par inoculation, à augmenter les rendements agronomiques (RDT), protéiques (RDN) ou unitaires (RUN) de la culture agronomique par rapport à un témoin dépourvu de ces mêmes microorganismes phytogènes.

4. Méthode de fertilisation-N raisonnée pour grandes cultures agronomiques selon une quelconque des revendications précédentes **caractérisée en ce que** le la transformation en score normalisé (Si) des différents indicateurs élémentaires permettant d'exprimer ces différents indicateurs élémentaires sur une même échelle ou dans une même unité et consistant à obtenir des classes, scores ou variables continues à partir de ces indicateurs élémentaires et de leurs valeurs de référence, est effectuée par ;

- classement
- notations discrètes sur une échelle fixe
- notations discrètes sur une échelle variable
- notations sur une échelle continue
- transformation en une unité de mesure commune
- normalisation interne

**5.** Méthode de fertilisation-N raisonnée pour grandes cultures agronomiques selon la revendication précédente **caractérisée en ce que** le la transformation en score normalisé (Si) des différents indicateurs élémentaires est calculé par rapport à une valeur choisie parmi un groupe non exhaustif comprenant ;

- à la valeur minimale ou maximale de l'indicateur avant normalisation,
- à la moyenne arithmétique, géométrique ou ordonnée de l'indicateur avant normalisation,
- à l'intégrale de Choquet ou la valeur de Sharpley associées à l'indicateur avant normalisation.

**6.** Méthode de fertilisation-N raisonnée pour grandes cultures agronomiques selon une quelconque des revendications précédentes **caractérisée en ce que** le poids, la pondération en sorte, Pi, attribué aux scores normalisés (Si) est calculé par une étape au cours de laquelle des poids sont attribués aux indicateurs élémentaires à agréger.

**7.** Méthode de fertilisation-N raisonnée pour grandes cultures agronomiques selon la revendication précédente **caractérisée en ce que** le poids, la pondération en sorte, Pi, attribué aux scores normalisés (Si) est calculé selon d'une des méthodes de calcul choisie parmi un groupe non exhaustif comprenant ;

- l'ACP (analyse par composante principale)
- l'AHP (*analytical hierarchy process*)
- l'opinion des acteurs de la fertilisation raisonnée

ou encore toutes autres méthodes statistiques ou de calcul qui de manière générale permettent la décomposition aux valeurs singulières par réduction d'un tableau comportant x variables sur y observations individuelles.

**8.** Méthode de fertilisation-N raisonnée pour grandes cultures agronomiques selon une quelconque des revendications précédentes **caractérisée en ce que** eMIC est *eAZB,* et que pMIC est *pAZB,* permettant ainsi la détermination d'*iAZB* en présence de RCS azotobactérisés, y compris par inoculation et réintroduction d'azotobactéries, de manière à ce que les valeurs d'eAZB estimées directement d'iAZB permettent elles aussi d'estimer dN comme suit ;

$$dN = \mathbf{a} \times pRDT - pAZB$$

sachant que a = [$b_{Arvalis}$ / $eAZB_{RUN}$] et pAZB = [pRDN - pRDN/$eAZB_{RDN}$]. pRDT et pRDN sont, respectivement, les objectifs, ou potentiels, de rendements agronomiques ($Qx_{grain}$/ha) et protéiques (kg-$N_{grain}$/ha), et $eAZB_{RDN}$ et $eAZB_{RUN}$ les valeurs d'eAZB fonction de RDN et RUN (rendements unitaires ; kg-Ngrain/uN fertilisant), a est le coefficient Arvalis ($b_{ARVALIS}$), *alias* « besoin unitaire » (uN/$Qx_{grain}$), divisé par $eAZB_{RUN}$, et pAZB représentant ici la contribution (ou fourniture) en N des azotobactéries résidusphériques, y compris celles réintroduites par l'inoculation des RCS, la formation de la dose d'engrais N et/ou P (dN) à partir de matières fertilisantes connues, sa préconisation sous forme d'apports fractionnés, et son épandage au champ étant eux pour le reste effectués de manières conventionnelles.

**9.** Méthode de fertilisation-N raisonnée pour grandes cultures agronomiques selon une quelconque des revendications précédentes **caractérisée en ce que** les engrais N sont des engrais à libération contrôlée (ELC, ou en anglais CRF ; *controlled release fertilizers*), ou encore des engrais à libération, ou dissolution, lente (EDL, ou en anglais SRF ; *slow release fertilizers*).

**Patentansprüche**

**1.** Eine Methode zur nachhaltigen N-Düngung von landwirtschaftlichem Ackerbau, die aus Schulung, Kalibrierung, Einteilung und Ausbringung von prognostizierten N-Düngermengen auf dem Feld besteht und folgende Schritte umfasst;

• die Bestimmung von **n elementaren Indikatoren** für die **mikrobielle Effizienz** (**eMIC**) von phytogenen Mikroorganismen des Systems Boden-Pflanze, einschließlich der durch Inokulation wiedereingeführten, zur Steigerung der landwirtschaftlichen Erträge (RDT), Proteinproduktion (RDN) oder Einheitserträge (RUN) der landwirtschaftlichen Kultur im Vergleich zu einer Kontroll-Kultur,

• die Aggregation dieser n elementaren Indikatoren (i) von eMIC, nach ihrer Umwandlung in ebenso viele normalisierte und gewichtete Punktzahlen, zu einem **aggregierten Indikator (iMIC)**, der also aus der Aggregation dieser *n* elementaren Indikatoren kommt, die aus den Boden- und Klimadaten der Ackerkrume gewonnen wurden,

• die Verknüpfung von eMIC mit iMIC,

• die Kalibrierung dieser N-Dünger-Dosen **(dN)** in Funktion von eMIC, empirisch abgeleitet von iMIC,

• abgewogen, Einteilung und Ausbringung von N-Dünger entsprechend dN

und in der;

die Aggregation der elementaren Indikatoren zu aggregierten Indikatoren, z. B. hier *iMIC,* erfolgt wie folgt;

$$iMIC = \sum (Si \times Pi) \text{ für die } i = 1, 2, 3 \ldots \rightarrow \text{Anzahl der Indikatoren}(n)$$

Wobei Si hier die normalisierte Punktzahl (Basis 100 oder 1,00) des elementaren Indikators ist, der eMIC beeinflusst, und *Pi* das Gewicht (0,00 bis 1,00) ist, das dieser Punktzahl zugewiesen wird, pMIC ist hier in Art der Beitrag (Bereitstellung) an N der phytogenen Mikroorganismen des Bodens, einschließlich jener durch die Inokulation der Kulturrückstände RCS wiedereingeführten,

und in der;

die genaue Einteilung und gewichtsmäßige Anwendung der N-Düngerdosis (dN) in situ in Abhängigkeit vom aggregierten Indikator iMIC, der seinerseits durch Aggregation der elementaren Indikatoren für die Fähigkeit der phytogenen Mikroorganismen des Bodens, einschließlich der durch Inokulation in das System Boden-Pflanze wiedereingeführten oder auch durch Anwendung von Signal- oder Biostimulanzien in situ stimulierten, zur Erhöhung von RDN und RUN erhalten wird, wobei RUN wie folgt berechnet wird;

$$dN = a \times pRDT - pMIC$$

wobei gilt, dass;

• $a = b_{ARVALIS} / eMIC_{RUN}$

• $pMIC = pRDN - pRDN/eMIC_{RDN}$ und dass pRDT und pRDN die Ziel- oder Potenzialwerte für die agronomischen ($Qx_{Korn}$/ha) und proteinhaltigen (kg-$N_{Korn}$/ha) Erträge sind, $eMIC_{RDN}$ und $eMIC_{RUN}$ die Werte von eMIC in Bezug auf RDN und RUN (Einheitserträge; kg-$N_{Korn}$/Einheit N-Dünger), jeweils und im Vergleich zu einer unbehandelten Kontrolle, und dass a der Koeffizient b ist, der sogenannte Arvalis™ ($b_{ARVALIS}$)-Koeffizient, alias "Einheitsbedarf" (d.h. N-Einheiten pro $Qx_{Korn}$), dividiert durch $eMIC_{RUN}$, wobei pMIC in diesem Fall der N-Beitrag (Bereitstellung) der phytogenen Mikroorganismen der Residuensphäre ist, einschließlich derjenigen, die durch Inokulation wieder eingeführt wurden, wobei die Bildung der N- und/oder P-Düngerdosis aus anerkannten Düngemitteln, ihre Einteilung in Form von geteilten Gaben und ihre Ausbringung auf dem Feld ansonsten auf herkömmliche Weise erfolgt.

2. Eine Methode zur nachhaltigen N-Düngung von landwirtschaftlichem Ackerbau, nach dem vorhergehenden Anspruch, **dadurch gekennzeichnet, dass** die elementaren Indikatoren von eMIC, die zu aggregierten oder zusammengesetzten Indikatoren zusammengefügt werden, die durch Probenahme der Ackerkrume von landwirtschaftlichen Parzellen erhalten werden, aus einer Gruppe ausgewählt werden, die Folgende umfasst;

• iAPC, d.h. ein elementarer Indikator für den Grad der agro-pedo-klimatischen Spezifität der azotobakteriellen Biomasse einer Bodenprobe von jedem einzelnen Acker;

• iRES, d.h. ein elementarer Indikator für die Widerstandsfähigkeit der bakteriellen Mikroflora eines Bodens in Bezug auf Vielfalt, Aktivität und/oder Abundanz bei Veränderungen des pH-Wertes des Bodens;

• iREC, d.h. ein elementarer Indikator für die Kohlenstoffeffizienz (eC) der tellurischen Mikroflora, insbesondere der Azotobakterien, in der Nähe von RCS (zellulosehaltige Residuen);

• iDAM, d.h. ein elementarer Indikator für den diazotrophen Zustand des Bodens, der eine Erhebung des RAT (herbstlicher Rest des mineralischen Stickstoffs) und einen Indikator für die diazotrophe Aktivität von Bodenbakterien umfasst.

• iVAZ, d. h. ein elementarer Indikator für die Aktivität der Azotobakterien im Boden, der die Menge an RCS (Residuen) und deren N-Gehalte sowie die Werte RVN = [ RCS / Nrcs ] und rMS = [ RCS / RAC ] integriert;
• iPAZ, d. h. ein elementarer Indikator für die Menge an P-Dünger, den die Azotobakterienflora in der Rückstandssphäre benötigt, um in landwirtschaftlicher Hinsicht optimal zu funktionieren.

3. Eine Methode zur nachhaltigen N-Düngung von landwirtschaftlichem Ackerbau, nach einem der vorhergehenden Ansprüche, **dadurch gekennzeichnet, dass** die Umwandlung der n verschiedenen elementaren Indikatoren (i) von eMIC in ebenso viele normalisierte Punktzahlen (Si) mithilfe von n Funktionen der Punkte-Zuordnung (alias FAS, oder Funktion der Nutzwerte) erfolgt, die diese elementaren Indikatoren beschreiben, die mit der mikrobiellen Effizienz (eMIC) phytogener Mikroorganismen des Boden-Pflanzen-Systems korrelieren, einschließlich der durch Inokulation wiedereingeführten Mikroorganismen, um die landwirtschaftlichen Erträge (RDT), Proteinerträge (RDN) oder Einheitserträge (RUN) der landwirtschaftlichen Kultur im Vergleich zu einer Kontrollkultur ohne die selben phytogenen Mikroorganismen zu steigern.

4. Eine Methode zur nachhaltigen N-Düngung von landwirtschaftlichem Ackerbau, nach einem der vorhergehenden Ansprüche, **dadurch gekennzeichnet, dass** die Umwandlung der verschiedenen elementaren Indikatoren in eine normalisierte Punktzahl (Si), es ermöglicht, diese verschiedenen elementaren Indikatoren auf einer selben Skala oder in einer gleichen Einheit auszudrücken, und die darin besteht, ausgehend von diesen elementaren Indikatoren und ihren Referenzwerten Klassen, Punktzahlen oder kontinuierliche Variablen zu erhalten, durchgeführt wird durch;

• Klassifizierung
• diskrete Bewertungen auf einer fixen Skala
• diskrete Bewertungen auf einer variablen Skala
• Bewertungen auf einer kontinuierlichen Skala
• Umwandlung in eine gemeinsame Maßeinheit
• interne Normalisierung

5. Eine Methode zur nachhaltigen N-Düngung von landwirtschaftlichem Ackerbau, nach dem vorhergehenden Anspruch, **dadurch gekennzeichnet, dass** die Umwandlung in eine normalisierte Punktzahl (Si) der verschiedenen elementaren Indikatoren in Bezug auf einen Wert berechnet wird, der aus einer nicht erschöpfend aufgezählten Gruppe ausgewählt wird, die Folgende umfasst;

• auf den minimalen oder maximalen Wert des Indikators vor der Normalisierung,
• auf das arithmetische, geometrische oder geordnete Mittel des Indikators vor der Normalisierung,
• auf das Choquet-Integral oder den Sharpley-Wert, die mit dem Indikator vor der Normalisierung verbunden sind.

6. Eine Methode zur nachhaltigen N-Düngung von landwirtschaftlichem Ackerbau, nach einem der vorhergehenden Ansprüche, **dadurch gekennzeichnet, dass** das Gewicht, die Gewichtung nach der Art, Pi, die den normalisierten Scores (Si) zugewiesen wird, durch einen Schritt berechnet wird, in dessen Verlauf den zu aggregierenden elementaren Indikatoren Gewichte zugewiesen werden.

7. Eine Methode zur nachhaltigen N-Düngung von landwirtschaftlichem Ackerbau, nach dem vorhergehenden Anspruch, **dadurch gekennzeichnet, dass** das Gewicht, die Gewichtung nach der Art, Pi, die den normalisierten Punktzahlen (Si) zugewiesen wird, nach einer der Berechnungsmethoden berechnet wird, die aus einer nicht erschöpfend aufgezählten Gruppe ausgewählt wird, die Folgende umfasst;

• die ACP (Analyse nach Hauptkomponenten)
• den AHP (*analytical hierarchy process*)
• die Meinung der Akteure im Bereich der nachhaltigen Düngung

oder auch alle anderen statistischen oder Rechenmethoden, die im Allgemeinen die Singulärwertzerlegung durch Reduktion einer Tabelle mit x Variablen auf y Einzelbeobachtungen ermöglichen.

8. Eine Methode zur nachhaltigen N-Düngung von landwirtschaftlichem Ackerbau, nach einem der vorhergehenden Ansprüche, **dadurch gekennzeichnet, dass** eMIC **eAZB** ist, und dass pMIC **pAZB** ist, wodurch die Bestimmung von **iAZB** in Gegenwart von mit Azotobakterien versetzten Residuen, einschließlich durch Inokulation und Wiedereinführung von Azotobakterien, ermöglicht wird, so dass die direkt aus iAZB geschätzten Werte von eAZB ihrerseits die Schätzung von dN wie folgt ermöglichen;

$$dN = \mathbf{a} \times pRDT - pAZB$$

wobei a = [$b_{Arvalis}$ / $eAZB_{RUN}$] und pAZB = [pRDN - pRDN/$eAZB_{RDN}$]. pRDT und pRDN sind, jeweils, die Ziele oder die Potenziale für agronomische Erträge ($Qx_{Korn}$/ha) und Proteinerträge (kg-$N_{Korn}$/ha), und $eAZB_{RDN}$ und $eAZB_{RUN}$ die Werte von eAZB in Abhängigkeit von RDN und RUN (Einheitserträge; kg-$N_{Korn}$/uN Dünger), **a** ist der Arvalis-Koeffizient ($b_{ARVALIS}$) *alias* "Einheitsbedarf" (uN/$Qx_{Korn}$), geteilt durch $eAZB_{RUN}$, und pAZB steht hier für den N-Beitrag (oder die N-Bereitstellung) der Azotobakterien der Residuensphäre, einschließlich der durch die Inokulation von Residuen wiedereingeführten, die Formung der Dosis von N- und/oder P-Dünger (dN) aus anerkannten Düngemitteln, ihre Einteilung auf Teilgaben und ihre Ausbringung auf dem Feld werden auf konventionelle Art und Weise durchgeführt.

9. Eine Methode zur nachhaltigen N-Düngung von landwirtschaftlichem Ackerbau, nach einem der vorhergehenden Ansprüche, **dadurch gekennzeichnet, dass** die N-Düngemittel Düngemittel mit kontrollierter Freisetzung (ELC, bzw. in engl. CRF; *controlled release fertilizers)* oder Düngemittel mit langsamer Freisetzung oder langsamer Auflösung (EDL, bzw. in engl. SRF; *slow release fertilizers*) sind.

**Claims**

1. Integrated N fertilization method for field-crops comprising the bulking, calibration, recommendation and field application of recommended doses of fertilizer N that includes the following steps;

   • the determination of *n elementary indicators* of *microbial efficiency* (*eMIC*) of phytogenic microorganisms in the soil-plant system, including those reintroduced by inoculation, to increase the agronomic (RDT), protein (RDN) or unit (RUN) yields of the agronomic crop compared to a control,
   • the aggregation of these n indicators (i) elementary eMIC, after their transformation into as many standardized and weighted scores, into an *aggregated indicator (iMIC)* thus resulting from the aggregation of these n elementary indicators obtained from the pedoclimatic data of the topsoil,
   • relating eMIC to iMIC,
   • the calibration of these doses of fertilizer-N (*dN*) is a function of eMIC thus empirically deduced from iMIC,
   • weighing, recommending, and spreading N-fertilizers corresponding to dN

   and in which ;
   the aggregation of elementary indicators into aggregated indicators, for example here *iMIC,* is accomplished as follows ;

   $$iMIC = \sum(Si \times Pi) \text{ for } i = 1, 2, 3 \dots \rightarrow \text{number of indicators } (n)$$

   Si being here the standardized score (base 100 or 1.00) of the elementary indicator affecting eMIC, and Pi the weight (0.00 to 1.00) attributed to this score, pMIC is here the contribution (supply) in N of the phytogenic microorganisms of the soil, including those reintroduced by the inoculation of the RCS,
   and in that ;
   the precise recommendation and in situ field application of the fertilizer N (dN) dose according to the aggregated indicator, iMIC, itself obtained by aggregation of elementary indicators soil-borne phytogenic microorganisms ability, including those reintroduced by inoculation to the soil-plant system, or stimulated in situ by application of signaling or bio-stimulating molecules, to increase RDN and RUN being calculated as follows ;

   $$dN = a \times pRDT - pMIC$$

   knowing that;

   • a = $b_{ARVALIS}$ / $eMIC_{RUN}$
   • pMIC = pRDN - pRDN/$eMIC_{RDN}$ and that pROT and pRDN are the objectives, or potentials, of agronomic

($Qx_{grain}$/ha) and protein (kg-$N_{grain}$/ha) yields, $eMIC_{RDN}$ and $eMIC_{RUN}$ the values of eMIC in terms of RDN and RUN (unit yields; kg-$N_{grain}$/unit of fertilizer N), respectively and in relation to an untreated control, and that a is the coefficient b said of Arvalis™ ($b_{ARVALIS}$ ), alias 'nitrogen requirement' (i.e. units of N by $Qx_{grain}$), divided by $eMIC_{RUN}$, pMIC being here the contribution (supply) in N of phytogenic residuspheric microorganisms, including those reintroduced by inoculation, the formation of the dose of fertilizers N and / or P from known fertilising materials, its recommendation in the form of fractional inputs, and its application to the field being for the rest carried out conventionally.

2. Integrated N fertilization method for field-crops according to the preceding claim **characterized in that** the elementary indicators of eMIC aggregated in aggregated or composite indicators obtained by sampling the topsoil of agricultural plots are selected from a group comprising ;

• iAPC, i.e. a basic indicator of the level of agro-pedoclimatic specificity of the azotobacterial biomasses of a soil sample from one to one agricultural plot;
• iRES, i.e. an elementary indicator of the level of resilience of the bacterial microflora of a soil in terms of diversity, activity and/or abundance faced with variations in soil pH;
• iREC, i.e. an elementary indicator of the carbon efficiency (eC) of telluric microflora, in particular azotobacterial, in the vicinity of the RCS;
• iDAM, i.e. a basic indicator of the diazotrophic state of the soil comprising only a survey of THE TTs and an indicator of diazotrophic activity of soil bacteria.
• iVAZ, i.e. an elementary indicator of the activity of soil azotobacteria integrating the amount of RCS and their N contents, as well as the values RVN = [ RCS / Nrcs ] and rMS = [ RCS / RAC ];
• iPAZ, i.e. an elementary indicator of the amount of P fertilizer needed by the *residusphere* azotobacterial flora to function optimally in agronomic terms.

3. Integrated N fertilization method for field-crops according to any of the preceding claims **characterized in that** the transformation of the n different elementary indicators (i) of eMIC into as many standardized scores (If) this fact using n scoring functions (aka FAS, or utility function) describing these elementary indicators correlated with the microbial efficiency (eMIC) of phytogenic microorganisms in the soil-plant system, including those reintroduced by inoculation, to increase the agronomic (RDT), protein (RDN) or unit (RUN) yields of the agronomic crop compared to a control without these phytogenic microorganisms.

4. Integrated N fertilization method for field-crops according to any of the preceding claims **characterized in that** the transformation into a standardized score (Si) of the different elementary indicators allowing to express these different elementary indicators on the same scale or in the same unit and consisting in obtaining classes, scores or continuous variables from these elementary indicators and their reference values, is carried out by;

• ranking
• discrete notations on a fixed scale
• discrete notations on a sliding scale
• ratings on a continuous scale
• transformation into a common unit of measurement
• internal standardization

5. Integrated N fertilization method for field-crops according to the preceding claim **characterized in that** the transformation into a standardized score (Si) of the various elementary indicators is calculated in relation to a value chosen from a non-exhaustive group comprising ;

• the minimum or maximum value of the indicator before standardisation,
• the arithmetic, geometric or ordered mean of the indicator before normalization,
• to the Choquet integral or Sharpley value associated with the indicator before normalization.

6. Integrated N fertilization method for field-crops according to any of the preceding claims **characterized in that** the weight, the weighting in kind, Pi, assigned to the standardized scores (Si) is calculated stepwise using weights assigned to the elementary indicators to be aggregated.

7. Integrated N fertilization method for field-crops according to the preceding claim **characterized in that** the weight, the weighting in kind, Pi, assigned to the standardized scores (Si) is calculated according to one of the methods of calculation chosen from a non-exhaustive group comprising ;

- CPA (principal component analysis)
- AHP (analytical hierarchy process)
- integrated fertilizer management advocate opinion

or any other statistical or calculation methods which generally allow the decomposition to singular values by reducing a table comprising x variables on y individual observations.

8. Integrated N fertilization method for field-crops according to any of the preceding claims **characterized in that** eMIC is *eAZB,* and that pMIC is *pAZB,* thus allowing the determination of *iAZB* in the presence of azotobacteria treated RCS, via inoculation and reintroduction of azotobacteria, so that the values of eAZB estimated directly from iAZB also allow to estimate dN as follows;

$$dN = \mathbf{a} \times pRDT - pAZB$$

knowing that a = $[b_{Arvalis} / eAZB_{RUN}]$ and pAZB = $[pRON - pRDN/eAZB_{RDN}]$. pRDT and pRDN are, respectively, the objectives, or potential, of agronomic yields ($Qx_{grain}$/ha) and protein yields (kg-$N_{grain}$/ha), and $eAZB_{RDN}$ and $eAZB_{RUN}$ the values of eAZB function of RDN and RUN (unit yields; kg-$N_{grain}$/uN-fertilizer), **a** is the Arvalis coefficient ($b_{ARVALIS}$), *alias* "nitrogen requirement" (uN/$Qx_{grain}$ ), divided by $eAZB_{RUN}$, and pAZB representing here the contribution (supply) in N of the *residusphere* azotobacteria, including those reintroduced by the inoculation of the RCS, the bulking of the N and/or P (dN) fertilizer dose (dN) using known fertilizer materials, its recommendation in the form of split applications in the field being for the rest carried out in conventional ways.

9. Integrated N fertilization method for field-crops according to any of the preceding claims **characterized in that** fertilizers N are fertilizers with controlled release (CRF; *controlled release fertilizers*), or slow-release fertilizers (SRF; *slow-release fertilizers*).

Figure 1

Figure 2

Figure 3

Figure 4

Figure 5

**Protéine du grain en % MS (RDT)**

Figure 6

**Rendement unitaire (RUN ; uN/uN)**

Figure 7

**Reliquats Nm post-récolte (RPR) en % dX ou dN**

Figure 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2223586 A
- US 5668719 A
- EP 2845906 A
- EP 13366004
- EP 2684588 A
- EP 13366005
- EP 2728353 A

- EP 13366006
- EP 2730926 A
- EP 15290111
- EP 3120679 A
- EP 16179846
- EP 16179850
- EP 3120680 A

**Littérature non-brevet citée dans la description**

- **ANDREWS ; SUSAN S ; JEFFREY P ; MITCHELL ; ROBERTO MANCINELLI ; DOUGLAS L. KARLEN ; TIMOTHY K. HARTZ ; WILLIAM R. HORWATH ; G. STUART PETTYGROVE ; KATE M. SCOW.** On-Farm Assessment of Soil Quality in California's Central Valley. *Agron. J.,* 2002, vol. 94, 12-23
- **ANDREWS ; SS. DL. KARLEN ; CA. CAMBARDELLA.** The Soil Management Assessment Framework : A Quantitative Soil Quality Evaluation Method. *Soil Sci. Soc. Am. J,* 2004, vol. 68, 1945-1962
- **ARVALIS.** b variétaux » : besoin en azote au quintal - Perspectives. *Institut du végétal / COMIFER,* 2014
- **ARVALIS.** Gérer la fertilisation du blé sans la méthode du bilan. *Réussir Grandes Cultures,* Novembre 2016, (307), 32-34
- **BASTIDA, F ; A. ZSOLNAY ; T. HERNÁNDEZ ; C. GARCÍA.** Past, présent and future of soit quality indicateurs: A biological perspective. *Geoderma,* 2008, vol. 147, 159-171
- **BISSONNAIS ; LE Y ET SOUDER ; LE C.** Mesurer la stabilité structurale des sols pour évaluer leur sensibilité à la battance et à l'érosion. *Étude et Gestion des Sols,* 1995, vol. 2 (1), 43-56
- Conceptual basis, formalisations and parameterization of the STICS crop model. 2008
- Méthode d'aide à la décision multi-critières pour l'internalisation/externalisation ''durable. **BOUKHERROUB ; TASSEDA ; ALAIN GUINET ; JULIEN FONDREVELLE.** 9th International Conférence on Modeling, Optimization & SIMulation. Juin 2012

- **CINELLI ; MARCO ; STUART R COLES ; KERRY KIRWAN.** Analysis of the potentiels of multi criteria décision analysis methods to conduct sustainability assessment. *Ecological Indicators,* 2014, vol. 46, 138-148
- **CLAUDE, P.-P. ; MACKERUIE, A. F ; COULMAN, B. E ; MEHUYS, G. R.** Effet du labour printanier et des cultures intercalaires de trèfle rouge sur le rendement du maïs-grain. *Can. J. Plant Sci.,* 1993, vol. 73, 37-46, http://www.nrcresearchpress.com/doi/pdfplus/10.4141/cjps93-006
- **CLAUDE, P-P ; M. GIROUX.** Effet des engrais organo-minéraux inoculés (EOMI) sur la croissance des plants de maïs-grain, les rendements, les prélèvements des éléments nutritifs et la qualité des grains. *Agrosol,* 2006, vol. 17, 51-64, https://www.irda.qc.ca/assets/documents/Publications/documents/claude-giroux-2006 article effet eomi mais.pdf
- **CLAUDE, P.-P. ; L. FILLION.** Effet de l'apport d'un inoculum bactérien aux résidus de culture de maïs-grain au sol sur le rendement et la qualité de blés d'hiver panifiables en France. *Agrosol,* 2004, vol. 15, 23-29, http://collections.banq.qc.ca/ark:/52327/bs604022
- Cultures annuelles et prairies. Calcul de la fertilisation azotée : Guide méthodologique pour l'établissement des prescriptions locales. Avril 2011
- **DUMANSKI, J. ; W.W. PETTAPIECE ; D.F. ACTON ; P-P. CLAUDE.** Application of agro-ecological concepts and hierarchy theory in the design of databases for spatial and temporal characterisation of land and soil. *Geoderma,* 1993, vol. 60, 343-358, http://www.sciencedirect.com/science/article/pii/001670619390Q35J

- **FELSKE, A ; A.D.L. AKKERMANS.** Spatial Homogeneity of Abundant Bacterial 16S rRNA Molécules in Grassland Soils. *Microb Ecol,* 1998, vol. 36, 31-36
- **GLOVER, JD ; JP REGANOLD ; PK ANDREWS.** Systematic method for rating soit quality of conventional, organic, and integrated apple orchards in Washington State. *Agriculture, Ecosystems and Environment,* 2000, vol. 80, 29-45
- **HORWATH ; WILLIAM R ; G. STUART PETTYGROVE ; KATE M. SCOW ; DANIEL S. MUNK.** On-Farm Assessment of Soil Quality in California's Central Valley. *Agron. J,* 2002, vol. 94, 12-23
- **JUSTES, E ; B MARY ; JM MEYNARD ; JM MACHET ; L THELIER-HUCHE.** Determination of a critical nitrogen dilution curve for winter wheat crops. *Ann. Bot.,* 1994, vol. 74, 397-407
- **KNOEPP, JD ; DC. COLEMAN ; DA. CROSSLEY JR ; JS. CLARK.** Biological indicateurs of soil quality: an ecosystem case study of their use. *Forest Ecology and Management,* 2000, vol. 138, 357-368
- **LAIREZ, J ; P FESCHET ; J AUBIN ; CH BOSKSTALLER ; I BOUVAREL.** Agriculture et développement durable : guide pour l'évaluation multicritère. 2015
- **LEBART, L ; A MORINEAU ; M PIRON.** Statistiques exploratoire multidimensionnelles. 2000
- **MORVAN, T ; L. BEFF ; Y. LAMBERT.** Minéralisation de l'azote des sols (Ouest) : résultats du projet "Mh. *Rencontres de la fertilisation raisonnée COMIFER 2015,* 18 Novembre 2015
- **MULVANEY, RL ; S. A. KHAN ; T. R. ELLSWORTH.** Synthetic Nitrogen Fertilizers Deplete Soil Nitrogen: A Global Dilemma for Sustainable Cereal Production. *J. Environ. Qual,* 2009, vol. 38, 2295-2314
- **RAVIER, CLÉMENCE ; JEAN-MARC MEYNARD ; JEAN-PIERRE COHAN ; PHILIPPE GATE ; MARIE-HÉLÈNE JEUFFROY.** Early nitrogen deficiencies favor high yield, grain protein content and N use efficiency in wheat. *European Journal of Agronomy,* 2017, vol. 89, 16-24
- **RAVIER, CLÉMENCE ; MARIE-HÉLÈNE JEUFFROY ; JEAN-MARC MEYNARD.** Mismatch between a science-based decision tool and its use: The case of the balance-sheet method for nitrogen fertilization in France. *NJAS - Wageningen Journal of Life Sciences xxx (2016) xxx-xxx (in press),* 2016
- **RAVIER, CLÉMENCE ; MARIE-HÉLÈNE JEUFFROY ; JEAN-MARC MEYNARD.** Les travaux des GREN, révélateurs de controverses autour de la méthode du bilan. *Rencontres de la fertilisation raisonnée COMIFER 2015,* 18 Novembre 2015
- **SAATY, THOMAS L.** Decision making with the analytic hierarchy process. *Int. J. Services Sciences,* 2008, vol. 1 (1), 2008-83
- **SCHLOTER, M ; O. DILLY ; JC MUNCHA.** Indicators for evaluating soil quality. *Agriculture, Ecosystems and Environment,* 2003, vol. 98, 255-262